# EUROPEAN PATENT SPECIFICATION

(11) **EP 3 962 887 B1**
(45) Date of publication and mention of the grant of the patent: **05.08.2026**
(21) Application number: 20705734.0
(22) Date of filing: 24.02.2020
(51) Int. Cl.: C07C 37/00, C07D 311/14, C07C 39/04, C07D 319/16, C07C 43/235, C07D 333/60, C07H 15/00, C07C 49/76, A61P 31/04, A61K 31/05

(54) **PHENOL DERIVATIVES FOR USE AS ANTIMICROBIAL, ANTIBACTERIAL, BACTERICIDE**
PHENOLDERIVATE ZUR VERWENDUNG ALS ANTIMIKROBIELLES ANTIBAKTERIELLES BAKTERIZID
DÉRIVÉS DE PHÉNOL DESTINÉS À ÊTRE UTILISÉS COMME ANTIMICROBIENS, ANTIBACTÉRIENS, ET BACTÉRICIDES

(30) Priority: 07.03.2019 IT 201900003343
(43) Date of publication of application: 09.03.2022
(73) Proprietor: Special Product's Line S.p.A., 00193 Roma (IT)
(72) Inventor: DALLAVALLE, Sabrina, 20871 Vimercate (MB) (IT); PISANO, Claudio, 04011 Aprilia (LT) (IT); FLORIO, Massimiliano, 00193 Roma (IT); CAMPONESCHI, Claudio, 00193 Roma (IT)
(74) Representative: Leone, Mario
(86) International application number: PCT/EP2020/054717
(87) International publication number: WO 2020/178053

(56) References cited:
- EP-A1- 3 301 085
- WO-A1-03/011808
- WO-A1-2016/097626
- WO-A1-2017/053778
- WO-A1-2018/213609
- KIM ET AL.: "A new class of synthetic retinoid antibiotics effective against bacterial persisters", NATURE, vol. 556, 5 April 2018 (2018-04-05), pages 103 - 107+12pp, XP002792197
- GARATTINI E ET AL: "ST1926, a novel and orally active retinoid-related molecule inducing apoptosis in myeloid leukemia cells: modulation of intracellular calcium homeostasis", BLOOD : JOURNAL OF THE AMERICAN SOCIETY OF HEMATOLOGY, AMERICAN SOCIETY OF HEMATOLOGY, US, vol. 103, no. 1, 2004, pages 194 - 207, XP002498487, ISSN: 0006-4971, [retrieved on 20030904], DOI: 10.1182/BLOOD-2003-05-1577
- CINCINELLI ET AL: "Synthesis and structure-activity relationships of new antiproliferative and proapoptotic retinoid-related biphenyl-4-yl-acrylic acids", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 15, no. 14, 2 June 2007 (2007-06-02), pages 4863 - 4875, XP022103873, ISSN: 0968-0896, DOI: 10.1016/J.BMC.2007.04.057
- MENG-HSIN CHEN ET AL: "BIOORGANIC & MEI)ICINAL CHEMISTRY LETTERS Pergamon Bioorganic & Medicinal Chemistry Letters 9 CARBOHYDROXAMIDO-OXAZOLIDINES: ANTIBACTERIAL AGENTS THAT TARGET LIPID A BIOSYNTHESIS", 1999, XP055687680, Retrieved from the Internet <URL:https://www.sciencedirect.com/journal/bioorganic-and-medicinal-chemistry-letters> [retrieved on 20200421]

## Description

### Field of the invention

The present invention relates to the field of pharmaceutical compounds, in particular phenol derivatives, also for use as antimicrobial, antibacterial, bactericide.

### State of the Art

The scientific publication Nature Wooseong Kim et al.,2018, A new class of synthetic retinoid antibiotics effective against bacterial persisters, 556, 103-107 doi:10.1038/nature26157, discloses the following compounds: 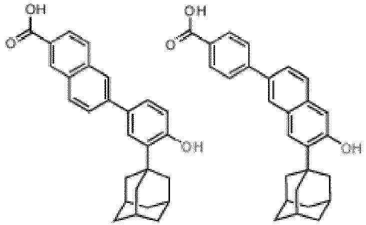 exhibiting high killing rates, synergism with gentamicin, and a low probability of resistance selection, in a C. elegans- MRSA killing assay against MRSA strain MW2 and activity against a panel of clinical S. aureus and Enterococcus faecium strains, but not against Gram-negative species.

WO9801132 and WO0156563 disclose adamantyl containing retinoid compounds used as anticancer agents.

International patent application N. WO2017053778 specifically discloses the following compounds:
6-(4-hydroxy-3-tricyclo[3.3.1.13,7]dec-1-ylphenyl)-2-naphthalenecarboxylic acid, 4-(6-hydroxy-7-tricyclo[3.3.1.13,7]dec-1-yl-2-naphthalenyl)-benzoic acid, (2E)-3-(4'-Hydroxy-3'-tricyclo[3.3.1.13,7]dec-1-yl[1,1'-biphenyl]-4-yl)-2-propenoic acid, pyridin-2-ylmethyl 9-fluoro-3-methyl-10-(4-methylpiperazin-1-yl)-7-oxo-3,7-dihydro-2H-[1,4]oxazino[2,3,4-ij]quinoline-6-carboxylate, 2,3,4,6-tetra-o-acetyl-1-thio-D-glucanpyranosato-S-(triethyl-phosphine)gold,
useful in treating bacterial infections such as a bacterial infection.

International patent application N. WO2018213609 discloses the preparation of the following compounds:
Methyl 6-(3-(adamantan-1-yl)-4-hydroxyphenyl)-2-naphthoate
2-(adamantan-1-yl)-4-(6-(hydroxymethyl)naphthalen-2-yl)phenol
6-(3-(adamantan-1-yl)-4-hydroxyphenyl)-2-naphthamide
6-(3-(adamantan-1-yl)-4-hydroxyphenyl)-N-ethyl-2-naphthamide
Methyl 6-(3-(adamantan-1-yl)-4-methoxyphenyl)-2-naphthoate
(6-(3-(adamantan-1-yl)-4-methoxyphenyl)naphthalen-2-yl)methanol
6-(3-(adamantan-1-yl)-4-methoxyphenyl)-2-naphthamide
6-(3-(adamantan-1-yl)-4-methoxyphenyl)-N-ethyl-2-naphthamide
6-(3-benzyl-4-hydroxyphenyl)-2-naphthoic acid
6-(5-(adamantan-1-yl)-2-hydroxyphenyl)-2-naphthoic acid
6-(3-(adamantan-1-yl)-4-hydroxyphenyl)-4-hydroxy-2-naphthoic acid
6-(3-benzyl-4-hydroxyphenyl)-4-hydroxy-2-naphthoic acid
6-(5-(adamantan-1-yl)-2-hydroxyphenyl)-4-hydroxy-2-naphthoic acid
6-(3-(adamantan-1-yl)-4-hydroxyphenyl)-4,8-dihydroxy-2-naphthoic acid
6-(3-benzyl-4-hydroxyphenyl)-4,8-dihydroxy-2-naphthoic acid
6-(5-(adamantan-1-yl)-2-hydroxyphenyl)-4,8-dihydroxy-2-naphthoic acid
and their antibacterial activity in particular against *Cutibacterium (Propionibacterium)* acnes bacteria.

International patent application, publication n. WO01/56554 discloses retinoid-type molecules for preparing compositions for preventive or curative treatment of bacterial colonisation, deterioration in pathological conditions caused by said colonisation and secondary skin infections induced by said bacteria and more particularly by the *Staphylococcus aureus.* WO01/56554 Specifically discloses the following compounds:
4-[4-(4'-Propyl-biphenyl-2-yl)-but-3-en-1-ynyl] benzoic acid
2-Hydroxy-4-(3,5,5,8,8-pentamethy)-5,6,7,8-tetrahydronaphthalen-2-ylselanylethynyl) benzoic acid
2'-(3-Methoxy-5,5,8,8-tetramethyl-5,6,7,8-tetrahydronaphthalen-2-yl)-[1,1',4', 11-terphenyl]-4-carboxylic
6-[Butoxy-(5,5,8,8-tetramethyl-5,6,7,8-tetrahydro-naphthalen-2-yl)-methyl]-naphthalene-2-carboxylic acid
4-[3-(1,1-Dimethyl-decyl)-4-methoxy-benzoylamino] benzoic acid
4-(3-Adamantan-1-yl-4-hexyloxy-benzoylamino) benzoic acid
643-(1-adamantyl)-4-hydroxyphenyl]-2-naphthylmethanol
2-Hydroxy-4-(5,5,8,8-tetramethyl-5,6,7,8-tetrahydro-anthracen-2-yl) -benzic acid
2-(3-Adamantan-1-yl-4-hydroxy-phenyl)-benzofuran-5-carboxylic acid
446-methoxyethoxymethoxy-7-(1-adamantyl)-2-naphthyl] salicylic acid
(E)-444-(5-Methoxymethoxy-4'-methyl-biphenyl-2-yl)-but-3-en-1-yl] benzoic acid
4-[4-(3-Methoxy-4'-methyl-biphenyl-2-yl -but-3-en-1-ynyl]-benzic acid
4-(3,5,5,8,8-Pentamethy)-5,6,7,8-tetrahydro-naphthalen-2-ylselanylethynyl)-benzic acid
2-Methoxymethoxy-2'-(5,5,8,8-tetramethyl-5,6,7,8-tetrahydronaphthalen-2-yl)-[1,1',4',1"] terphenyl-4"-carboxylic
2-Hydroxy-1-(5,5,8,8-tetramethyl-5,6,7,8-tetrahydronaphthalen-2-yl)-[1,1',4',1"]terphenyl-4-acid - carboxylic
4-[(2)-2-(5,6,7,8-tetrahydro-5,5,8,8-tetramethyl-2-naphthyl)-2-undecenamido] benzoic acid
444-(4'-Methyl-biphenyl-2-yl)-but-3-en-1-ynyl] benzoic acid
21-Propoxy-5'-(5,5,8,8-tetramethy)-5,6,7,8-tetrahydronaphthalen-2-yl) -biphenyl-4-carboxylic acid
4 [2-Heptyloxyimino-2-(5,5,8,8-tetramethy)-5,6,7,8-tetrahydronaphthalen-2-yl)acetylamino] benzoic acid6-(5,5,8,8-Tetramethy)-5,6,7,8-tetrahydro-naphthalen-2-yloxy)-naphthalene-2-carboxylic acid amide
6-(3-Adamantan-1-yl-4-methoxy-phenyl)-naphthalene-2-carboxylic acid (2-dimethylamino-ethyl)-amide
2-Hydroxy-4-(5,5,8,8-tetramethyl-5,6,7,8-tetrahydro-naphthalen-2-ylselanylethynyl)benzoic acid
2'-(3-Methoxymethoxy-5,5,8,8-tetramethyl-5,6,7,8-tetrahydronaphthalen-2-yl)-[1,1',4',1"] terphenyl-4"-carboxylic
3-Hydroxy-2'-(5,5,8,8-tetramethyl-5,6,7,8-tetrahydronaphthalen-2-yl)-[1,1',4',1"] terphenyl-4-acid carboxylic
3-(5'-Adamantan-1-yl-4'-methoxy-21-methyl-biphenyl-3-yl)-acrylic acid

Also WO2018213609 and EP3301085 disclose retinoid-type antibacterial compounds.

### Technical problem

Bacterial resistance to antimicrobial drugs might become one of the biggest threats to human health in our times. The increasing occurrence of infections with multi-drug-resistant pathogens is associated with high mortality and morbidity (H. W. Boucher et al. Clin. Infect. Dis., 2013, 56, 1685-1694) and the prevailing lack of new efficient antimicrobial drugs for treatment of these infections has led to serious concerns.

Bacterial resistance includes the modification or overexpression of the antibiotics target, the decrease of the intracellular antibiotic concentration, by either expression of efflux systems actively transporting the drug out of the cell or by mechanism that reduce their influx, and the expression of enzymes able to deactivate the antibiotic.

It is therefore clear that the need for novel antimicrobial drugs is a continuous one in order to counteract the development of bacterial resistance.

### Object of the invention

The technical problem is solved by providing compounds of formula (I) (named as compounds of class A) 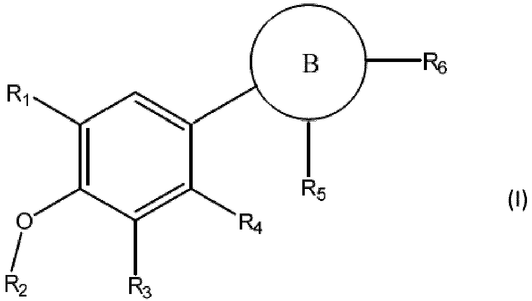 wherein
B is C₃-C₁₂ cycloakyl, or phenyl, or a C₃-C₁₃ heterocycle containing S,N,O
R1 is H, C₁-C₁₂ alkyl, C₁-C₁₂ alkenyl, C₁-C₁₂ alkynyl linear or branched, C₃- C₁₂ cycloalkyl optionally substituted with C₁-C₄ alkyl or OH, C₁-C₃ arylalkyl, phenyl, optionally substituted with C₁-C₄ alkyl, linear or branched, heterocycle containing at least one heteroatom selected from the group consisting of: N, O,S, optionally substituted with OH, NH₂
R2 is H, C₁-C₁₂ alkyl, C₁-C₁₂ alkenyl, C₁-C₁₂ alkynyl linear or branched, (CH₂)ₙ-R7, (CH₂)ₙO (CH₂)ₘO-(CH2) p-R8, (CH₂O)n CH₃, CO-R8, (CH₂)ₙ-R10, D-mannosyl, glucuronate, (SO₂)OH
R7 is OH, COOH, CONHO-R8, (CH₂)ₙO(CH₂)ₘO-R8, CN, NH₂,
R8 is H, C₁-C₁₂ alkyl, C₁-C₁₂ alkenyl, C₁-C₁₂ alkynyl linear or branched
R10 is NH₂, N-containing heterocycle, amidine, guanidine
R3 is H, OH, CHO or
R2 and R3 are linked to form a cycle comprising at least one atom of O and/or N
R4 is H
R5 is CH=NO-R14, CH=NO- (CH₂)ₙ-NH₂, (CH₂)ₙNHCO-R14,
R14 is C₁-C₅ alkyl C₁-C₅ alkenyl, C₁-C₅ alkynyl linear or branched, C₃- C₁₂ cycloakyl, aryl, C₁-C₃ arylalkyl, (CH₂)n-COOH
R4 and R5 can be joined together to form CH=CH or CO or O or S or SO or SO₂
R6 is =CHCOOH, (CH₂)ₙCH=NOH, =CHCOOR16, CX=CYCOOH, CX=CYCN, CX=CYCOO-R16, CX=CY-tetrazolyl, CX=CYCONH₂, CX=CY-CONH-R16, CX=CYCONH-aryl, CX=CYCONHOH, CX=CYCH₂OH, C≡CCOOH, C≡CCH2OH, C≡CCOO-R16, C≡CCONH₂, C≡CONH-R16, C=CCONH-aryl, C=CCONHOH, CX=CYCOOglycosyl, C=CCOOglycosyl,
R16 is H, C₂-C₅ alkyl, C₂-C₅ alkenyl, C₂-C₅ alkynyl linear or branched, C₃- C₁₂ cycloalkyl, aryl, C₁-C₃ arylalkyl, (CH₂)n-COOH
X and Y, the same or different, are H, C₂-C4 alkyl linear or branched, halogen, CN,
n is an integer from 1 to 8
with the proviso that when R14 is CH₃, tert-butyl or (CH2)COOH, R6 is not CH=CH-COOH
or a pharmaceutically acceptable salt thereof.

Are also object of the present invention, compounds belonging to group A for use as a medicament and compounds belonging to group A for use as antimicrobial and/or antibacterial and/or bactericide.

Object of the invention are pharmaceutical compositions comprising at least one compound of formula (II) belonging to group A as active ingredient and pharmaceutically acceptable adjuvants and/or vehicles and/or excipients.

### Detailed description of the invention

### Definitions

Within the meaning of the present invention antibacterial or bactericide means any compounds able to kill bacteria or suppresses their growth or their ability to reproduce.

Within the meaning of the present invention bacteria can be gram-positive bacteria and/or gram-negative bacteria, such as *Staphilococcus aureus, Enterococcus faecalis, Escherichia coli, Streptococcus thermophilus.*

Within the meaning of the present invention antimicrobial means any compound being germicides, antibiotics, antibacterials, antivirals, antifungals, antiprotozoals and antiparasites.

Within the meaning of the present invention alkyl means to linear or branched alkyl groups having from 1 to 18 carbon atoms or from 1 to 12 carbon atoms or from 1 to 5 carbon atoms or from 1 to 4 carbon atoms or from 2 to 5 carbon atoms.

Within the meaning of the present invention cycloalkyl means saturated carbocyclic group of 3 to 12 carbon atoms or of 3 to 6 carbon atoms, optionally substituted with C₁-C₄ alkyl or OH or NH₂. An aromatic group is not intended. The cycloalkyl group can comprise a single ring or multiple condensed rings.

Within the meaning of the present invention heterocycle means a saturated or partially unsaturated, but not aromatic, C₃-C₁₃ or C₃-C₁₂ ring or multiple condensed ring containing at least one heteroatom selected from the group consisting of N, O, S and optionally substituted with OH, NH₂.

Within the meaning of the present invention aryl means aromatic ring, for example phenyl, optionally substituted with C₁-C₃ alkyl or C₁-C₄ alkyl, OH, NH₂.

Within the meaning of the present invention monosaccharide residue means simple sugar of general formula CₙH₂ₙOₙ such as triose, tetrose, pentose, hexose, heptose etc.

Within the meaning of the present invention glycosyl means a radical from a saccharide by removal of hydroxyl group.

New synthesised compounds belonging to group A are of formula (I) 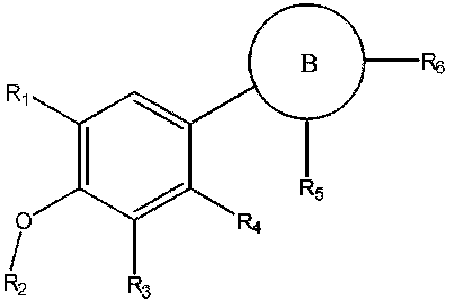 wherein
B is C₃-C₁₂ cycloalkyl, or phenyl, or a C₃-C₁₃ heterocycle containing S, N, O
R1 is H, C₁-C₁₂ alkyl C₁-C₁₂ alkenyl, C₁-C₁₂ alkynyl linear or branched, C₃-C₁₂ cycloalkyl optionally substituted with C₁-C₄ alkyl or OH, C₁-C₃ arylalkyl, phenyl, optionally substituted with C₁-C₄ alkyl, linear or branched, heterocycle containing at least one heteroatom selected from the group consisting of: N, O,S, optionally substituted with OH, NH₂
R2 is H, C₁-C₁₂ alkyl C₁-C₁₂ alkenyl, C₁-C₁₂ alkynyl linear or branched, (CH₂)ₙ-R7, (CH₂)ₙO(CH₂)ₘO-(CH₂)p-R8, (CH₂O)n CH₃, CO-R8, (CH₂)ₙ-R10, D-mannosyl, glucuronate, (SO₂)OH
R7 is OH, COOH, CONHO-R8, (CH₂)ₙO(CH₂)ₘO-R8, CN, NH₂,
R8 is H, C₁-C₁₂ alkyl C₁-C₁₂ alkenyl, C₁-C₁₂ alkynyl linear or branched
R10 is NH₂, N-containing heterocycle, amidine, guanidine
R3 is H, OH, CHO or
R2 and R3 are linked to form a cycle comprising at least one atom of O and/or N
R4 is H
R5 is CH=NO-R14, CH=NO- (CH₂)ₙ-NH₂, (CH₂)ₙNHCO-R14,
R14 is C₁-C₅ alkyl, C₁-C₅ alkenyl, C₁-C₅ alkynyl linear or branched, C₃- C₁₂ cycloakyl, aryl, C₁-C₃ arylalkyl, (CH₂)n-COOH
R4 and R5 can be joined together to form CH=CH or CO or O or S or SO or SO₂
R6 is =CHCOOH, (CH₂)ₙCH=NOH, =CHCOOR16, CX=CYCOOH, CX=CYCN, CX=CYCOO-R16, CX=CY-tetrazolyl, CX=CYCONH₂, CX=CY-CONH-R16, CX=CYCONH-aryl, CX=CYCONHOH, CX=CYCH₂OH, C≡CCOOH, C≡CCH₂OH, C≡CCOO-R16, C≡CCONH₂, C≡CONH-R16, C=CCONH-aryl, C=CCONHOH, CX=CYCOOglycosyl, C=CCOOglycosyl,
R16 is H, C₂-C₅ alkyl, C₂-C₅ alkenyl, C₂-C₅ alkynyl linear or branched, C₃- C₁₂ cycloakyl, aryl, C₁-C₃ arylalkyl, (CH₂)n-COOH
X and Y, the same or different, are H, C₂-C4 alkyl linear or branched, halogen, CN,
n is an integer from 1 to 8
with the proviso that when R14 is CH₃, tert-butyl or (CH2)COOH, R6 is not CH=CH-COOH
or a pharmaceutically acceptable salt thereof.

Preferably B is phenyl.

Preferably R1 is adamantyl, benzyl.

Preferably R2 is H.

Preferably R3 is H.

Preferably R5 is CH=NO-C(CH₃)₃, CH=NO-aryl; CH₂-1,4-Oxazinane

Preferably R6 is C≡CCOOH, C≡CC-CH₂OH, CH=CHCOOH, CH=CH-CH₂OH

More preferably the compounds belonging to group A are selected from the group consisting of:

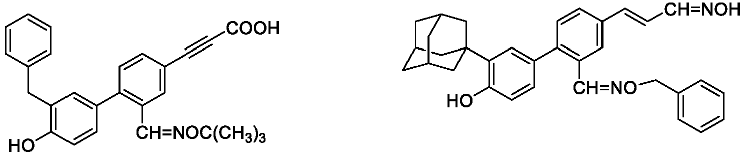

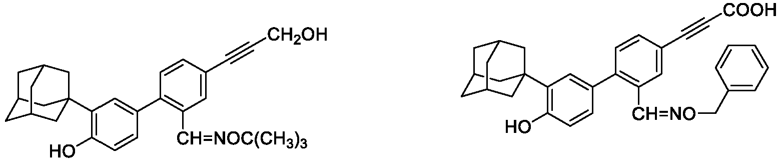

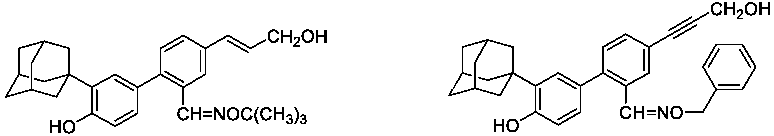

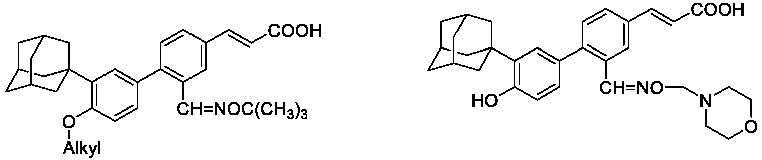

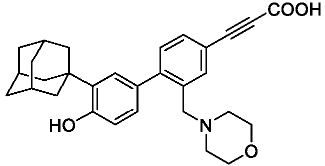

The pharmaceutical acceptable salts of the compound of formula (II) belonging to the group A are included in the scope of the invention.

Pharmaceutical acceptable salts are salts which retain the biological activity of the base and are derived from such known pharmacologically acceptable acids such as, e.g., hydrochloric, hydrobromic, sulfuric, phosphoric, nitric, fumaric, succinic, oxalic, malic, tartaric, maleic, citric, methanesulfonic or benzoic acid and others commonly used in the art, and their corresponding basis.

The compounds of the present invention can be synthesized by many methods available to those skilled in the art of organic chemistry. Examples of compounds of the present invention are given in the intermediates and examples section set out hereinafter.

Another object of the present invention is a pharmaceutical composition containing at least one compound of formula (II) of group A as an active ingredient, in an amount such as to produce a significant therapeutic effect, together with pharmaceutically acceptable vehicle and/or excipients.

The pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

### EXAMPLES

The Synthetized compounds from 1 to 15, are herein listed:

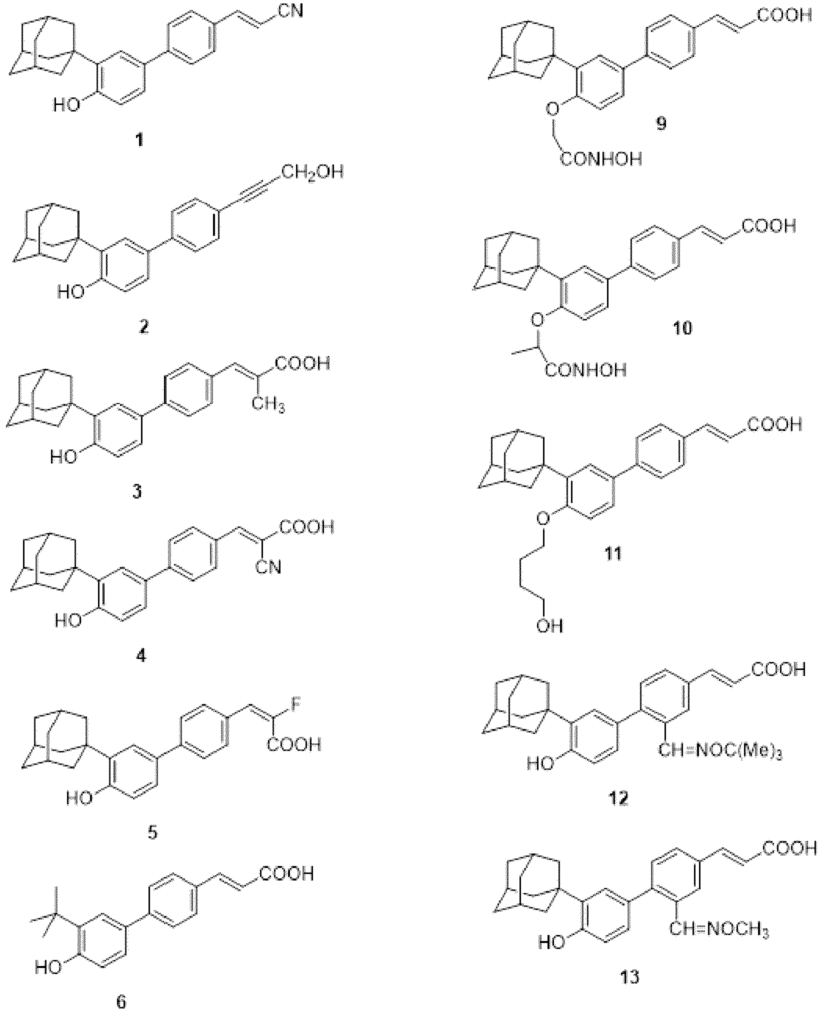

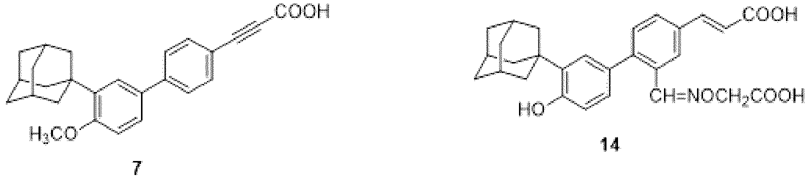

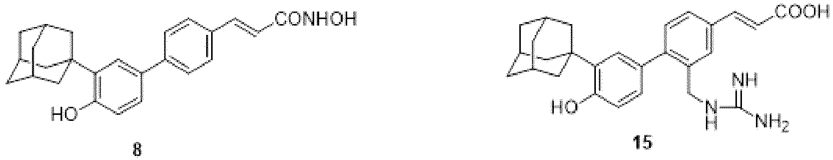

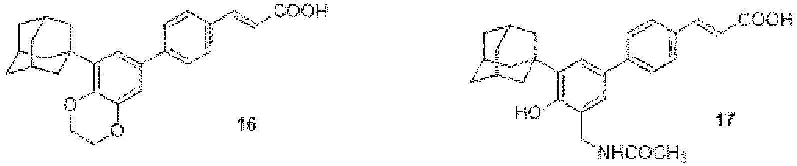

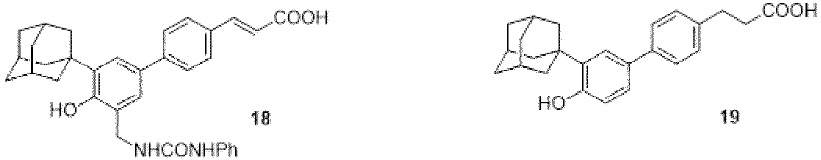

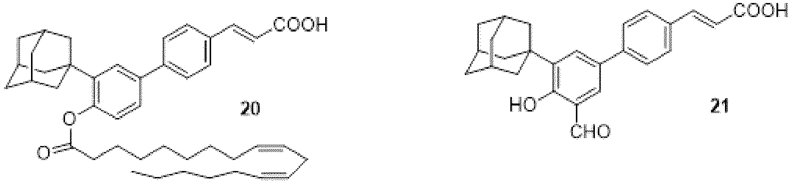

### Example 1: Synthesis of (E/Z)-3-(3'-(Adamantan-1-yl-4'-hydroxybiphenyl-4-yl)-acrylonitrile (1)

2-(Adamantan-1-yl)-4-(4-bromophenyl)phenol. A finely pulverized mixture of 4-(4-bromophenyl)phenol (200 mg, 0.8 mmol) and adamantan-1-ol (122 mg, 0.8 mmol) was added in portions and with stirring to 0.8 mL of a mixture of 9:1 AcOH/H₂SO₄. After 4 days at room temperature addition of ice/water and filtration gave 100% of the title compound, mp 140-143 °C, lit. 148-149 °C. ¹H NMR (CDCl₃) δ: 1.75 (6H, s, 6Ad), 2.03 (3H, s, 3Ad), 2.12 (6H, s, 6Ad), 6.70 (2H, d, 1Ar, J = 8.56 Hz),7.85 (1H, d, 1Ar, J = 8.56 Hz), 7.25 (1H, m, 1Ar), 7.30-7.42 (3H, m, 3Ar), 7.45 (2H, m, 2Ar) .

(E/Z)-3-(3'-(Adamantan-1-yl-4'-hydroxy-biphenyl-4-yl)-acrylonitrile (1). 2-(Adamantan-1-yl)-4-(4-bromophenyl)phenol . (300 mg, 0.782 mmol), 66.4 mg (1.25 mmol) of acrylonitrile, 8.78 mg (0.0391 mmol) of Pd(OAc)₂, and 23.80 mg (0.00782 mmol) of trio-tolylphosphine in 0.600 mL of Et3N were mixed in a round flask and heated at 100 °C for 8 h. Water and ice were added, 2 N HCl was added, and the aqueous phase was extracted with ethyl acetate. The organic phase was dried over Na2SO4, filtered, and evaporated. The crude product was purified by flash chromatography (hexane/ethyl acetate, 80:20) to give 124 mg of the product. (E/Z isomers, 2:1). Yield 45%. ¹H NMR (CDCl₃), E, δ: 1.78 (6H, s), 2.13 (3H, s), 2.17 (3H, s), 4.9 (1H, bs, OH), 5.87 (1H, d, J = 16.5 Hz), 6.72 (1H, d, J = 8.50 Hz), 7.30 (1H, dd, J = 8.50 Hz, J = 2.30 Hz), 7.41 (1H, d, J = 16.50 Hz), 7.45 (1H, d, J = 2.30 Hz), 7.47 (2H, d, J = 8.60 Hz), 7.58 (2H, d, J= 8.60 Hz). MS (EI) m/z: 355 (56, M+), 97 (100). ¹H NMR (CDCl₃), Z, δ: 1.78 (6H, s), 2.13 (3H, s), 2.17 (6H, s), 4.9 (1H, bs, OH), 5.40 (1H, d, J = 11.3 Hz), 6.72 (1H, d, J = 8.50 Hz), 7.13 (1H, d, J = 11.3 Hz), 7.33 (1H, dd, J = 8.50 Hz, J = 2.30 Hz), 7.48 (1H, d, J = 2.30 Hz), 7.62 (2H, d, J = 8.50 Hz), 7.85 (2H, d, J =8.50 Hz)

### Example 2: Synthesis of 3-(adamantan-1-yl)-4'-(3-hydroxyprop-1-yn-1-yl)-[1,1'-biphenyl]-4-ol (2)

2-(Adamantan-1-yl)-4-(4-bromophenyl)phenol (2 g, 5.22 mmol), was added to a mixture of CuI (0.017 g, 0.089 mmol), PdCl₂(Ph₃P)₂ ( 0.037g, 0.052 mmol, diisopropylamine (14.4 mol) and TEA (3.48 ml). The resulting mixture was stirred for 30 min at room temperature, added with propargyl alcohol (0.33g, 5.74 mmol), heated at 60 °C for 20 h. Evaporation, taking up with water and ice, addition of 2 N HCl, extraction with ethyl acetate and filtration of the exacts and evaporation, gave 2.47g of the crude compound. The product was purified by flash chromatography (hexane/ethyl acetate from 90:10 to 70:30) to give 347 mg of the title compound of the product. (19%). M.P. 200 °C. ¹H NMR (CDCl₃), δ: 1.80 (6H, s), 2.13 (3H, s), 2.17 (6H, s), 4.50 (1H, s), 5.40 (1H, d, J = 11.3 Hz), 6.69 (1H, d, J = 7.8 Hz), 7.27 (1H, dd, J = 7.8 Hz, J = 2.6 Hz), 7.37-7.55 (5H, m).

### Example 3: Synthesis of E-2-Methyl-3-[3'-(adamantan-1-yl)-4'-hydroxybiphenyl-4-yl]acrylic Acid (3)

A) 3'-(Adamantan-1-yl)-4'-tert-butyldimethylsilyloxybiphenyl- 4-aldehyde. 3-(Adamantan-1-yl)-4-(tert-butyldimethylsilyloxy) bromobenzene (1.56 g, 3.70 mmol) was dissolved in 7.5 mL of toluene. Then 3.7 mL of a 2 M aqueous solution of Na₂CO₃, 0.128 g (0.11 mmol) of tetrakistriphenylphosphine-palladium, and a solution of 610 mg (4.07 mmol) of 4-formylbenzeneboronic acid in 1.73 mL of ethanol were added. The mixture was refluxed for 2 h in a stream of nitrogen. It was then cooled, taken up with ethyl acetate, and washed with a NaCl saturated solution. The phases were separated, the organic layer was filtered, dried over Na₂SO₄, and filtered, the solvent was evaporated, and the residue was subjected to flash chromatography (hexane/ethyl acetate, 3:1) to give 1.09 g of the title compound, mp 158 °C.
B) E-2-Methyl-3-[3'-(adamantan-1-yl)-4'-hydroxybiphenyl-4-yl]acrylic Acid (3). Under a stream of nitrogen 200 mg (0.448 mmol) of 3'-(adamantan-1-yl)-4'-tert-butyldimethylsilyloxybiphenyl-4-aldehyde and 167 mg (0.448 mmol) of Ph₃Pd C(CH₃)-COOEt were dissolved in 2.3 mL of CHCl₃, and the resulting solution was refluxed for 21 h. The solvent was evaporated, and the crude ester obtained was used without further purification. This compound (390 mg, 0.714 mmol) was added to 90 mg (2.14 mmol) of LiOH.H₂O dissolved in 30 mL of THF/H₂O, 1:1. The solution was stirred at room temperature for 2 days. After evaporation of THF, the aqueous phase was acidified with 3 mL of 1 M HCl and extracted with AcOEt. Drying, filtration and evaporation of the solvent afforded 312 mg of crude product, which was purified by flash chromatography (hexane/ethyl acetate, 2:1 and then 3:2) to obtain 69 mg of the title compound as a white solid, mp 200 °C. ¹H NMR (600 MHz) (DMSO-d₆) δ 1.73-1.76 (6H, s, 6Ad), 2.04-2.06 (3H, m, 3Ad), 2.08 (Me, d, J = 1.59 Hz), 2.12-2.94 (6H, m, Ad), 6.86 (1H, d, H-5', J = 8.27 Hz), 7.36 (1H, dd, H-6', J = 2.38 Hz, J = 8.27 Hz), 7.38 (1H, d, H-2', J = 2.38 Hz), 7.51 (2H, d, H-3 and H-5, J =8.42 Hz), 7.60 (1H, br s, CH), 7.63 (2H, d, H-2 and H-6, d, J = 8.42 Hz) 9.50 (1H, s, OH), 12.4 (1H, s, COOH). MS (m/z): 388 (M+, 100), 267 (40), 178 (40), 79 (60).

### Example 4: Synthesis of 3-(3'-adamantan-1-yl)-4'-hydroxy-[1,1'-biphenyl]-4-yl)-2-cyanoacrylic acid (4)

A suspension of 3'-(Adamantan-1-yl)-4'-tert-butyldimethylsilyloxybiphenyl-4-aldehyde (Cincinelli et al J. Med. Chem. 2005, 48, 4931-4946) (150 mg, 0.34 mmol), methyl cyanoacetate (1g, 10.1 mmol) and beta-alanine (119 mg, 1.34 mmol) in ethanol (26 ml) was heated at 50 °C for 4 h. The solvent was evaporated and the yellow residue was taken up with CH₂Cl₂ (25 ml). The solution was washed three times with water (35 ml), dried and evaporated. The resulting solid was washed with ethanol to dissolve the starting cyanoacetate to give 151 mg (85%) of Methyl 3-(3'-adamantan-1-yl)-4'-tetrbutyldimethylsilyloxy-[1,1'-biphenyl]-4-yl)-2-cyanoacrylate M.p. 185 °C.

The above compound (100 mg, 0.19 mmol) was added to a solution (THF: H₂O 1:1, 7.7 ml) of LiOH.H2O (40 mg, 0.95 mmol). The mixture was stirred for 72 h at room temperature. THF was evaporated. The remaining aqueous phase was extracted with hexane, then acidified with 1N HCl to pH 2 and filtered to give 57 mg of a crude compound. Purification by reverse phase flash chromatography (MeOH: H2O 3:1) gave 26 mg of the title compound. M.p 228 °C ¹H NMR (acetone-d₆), δ: 1.78 (6H, s), 2.13 (3H, s), 2.17 (6H, s), 6.95-7.05 (1H, m), 7.40-7.50 (1H, m), 7.65-7.55 (1H, m), 7.77-7.90 (2H, m), 8.07-8.22 (2H, m),8.32 (1H, s),10.06 (1H, s).

### Example 5: Synthesis of E-2-Fluoro-3-[3'-(adamantan-1-yl)-4'-hydroxybiphenyl- 4-yl]-acrylic Acid (5)

E-2-Fluoro-3-[3'-(adamantan-1-yl)-4'-hydroxybiphenyl-4-yl]-acrylic acid (5). EtOOC-CH(F)-PO(OEt)₂ (124 mg, 0.493 mmol) was dissolved in 1 mL of anhydrous THF, cooled to -78 °C, and treated with a solution of 1.6 M BuLi in hexane (0.364 mL). After stirring for 30 min at -78 °C, an amount of 200 mg (0.448 mmol) of 3'-(adamantan-1-yl)-4'-tert-butyldimethylsilyloxybiphenyl-4-aldehyde (see example 3) dissolved in 0.5 mL of THF was dropped, and the solution was slowly brought to room temperature (3 h). After acidification with 6 mL of 2 N HCl the aqueous phase was extracted with AcOEt. The organic layers were washed with brine, dried, filtered, and evaporated to afford 280 mg of a crude product. Purification by flash chromatography (hexane/ethyl acetate, 95:5) gave 180 mg (75%) of the ethyl ester of the tertbutyldimethylsilyl derivative of the title compound as an oil. This compound (97 mg, 0.181 mmol) was suspended in a solution of 38 mg (0.905 mmol) of LiOH.H2O in 7.4 mL of THF/H₂O, 1:1, and stirred overnight at room temperature in the dark. THF was evaporated and the remaining aqueous phase was washed with hexane, then acidified with 0.5 mL of 2 N HCl. The light-yellow precipitate was filtered and dried to afford 55 mg of the title compound. Yield 77%, mp 202 °C. ¹H NMR (acetone-d₆): δ 1.8 (8H, s, 8 Ad), 2.2 (6H, s, 6 Ad), 6.90 (1H, d, J = 8.5 Hz), 7.00 (1H, d, JH,F = 23.9 Hz), 7.36 (1H, dd, J = 8.5 and 2.6 Hz), 7.50 (1H, d, J = 2.6 Hz), 7.60 (2H, d, J = 8.8 Hz), 7.71 (2H, d, J ) 8.8 Hz). MS (EI) m/z: 392 (M+, 100).

### Example 6: Synthesis of E-3-(3'-tert-Butyl-4'-hydroxybiphenyl-4-yl)acrylic Acid (6)

E-3-(3'-tert-Butyl-4'-hydroxybiphenyl-4-yl)acrylic acid (6). To a solution of CH3COOH/conc. H₂SO₄, 9:1 (2 mL), an amount of 500 mg (2.01 mmol) of 4-(4'-bromophenyl)-phenol was added. Then an amount of 0.192 mL (2.01 mmol) of tert-butyl alcohol was added dropwise. The mixture was stirred at room temperature for 2 weeks. CH₃COOH was evaporated, and water and ice were added. The white solid was filtered and washed with water. The crude product was purified by flash chromatography (hexane/ethyl acetate, 85: 15) to give 163 mg of 2-tert-butyl-4-(4'-bromophenyl) phenol. Yield 27%, mp 114-116 °C. The above compound (160 mg, 0.524 mmol), 72 mg (0.838 mmol) of methyl acrylate, 5 mg (0.0223 mmol) of Pd(OAc)₂, and 27 mg (0.0887 mmol) of tri-o-tolylphosphine in 0.243 mL of Et₃N were mixed in a round flask and heated at 100 °C for 4 h. Water and ice were added, 2 N HCl was added, and the aqueous phase was extracted with ethyl acetate. The organic phase was dried over Na₂SO₄, filtered, and evaporated. The crude product was purified by flash chromatography (dichloromethane/hexane, 95:5), to give 121 mg of methyl E-3-(3'-tert-butyl-4'-hydroxybiphenyl-4-yl)acrylate. Yield 74%, mp 182 °C. The above compound (70 mg, 0.226 mmol) was dissolved in a solution of 47.4 mg (1.13 mmol) of LiOH.H2O in 9.30 mL of THF/H₂O, 1:1, and the solution was stirred overnight at room temperature in the dark. THF was evaporated. The aqueous phase was washed with hexane and Et2O and then acidified with 2 N HCl (0.6 mL). The white solid was filtered and dried to give 54 mg of pure product. Yield 81%, mp 218 °C. ¹H NMR (DMSO-d₆) δ 1.34 (9H, s, 3CH3), 6.47 (1H, d, CH, J = 16.38 Hz), 6.83 (1H, d, 1Ar, J = 8.19 Hz), 7.34 (1H, dd, 1Ar, J = 8.19 Hz, J = 2.23 Hz), 7.40 (1H, d, 1Ar, J = 2.23 Hz), 7.55 (1H, d, CH, J = 16.38 Hz), 7.58 (2H, d, 2Ar, J = 8.25 Hz), 7.68 (2H, d, 2Ar, J = 8.25 Hz), 9.63 (1H, s, OH). MS (EI) m/z: 296 (97, M+), 281 (100), 253 (64).

### Example 7: Synthesis of 4-(3-(1-adamantvl)-4-methoxyphenyl)propiolic acid (7)

A) *Methyl 4-(3-(1-adamantvl)-4-methoxvphenyl)propiolate.* 301 mg (1.26 mmol) of methyl 4-bromophenylpropiolate were dissolved in 2.5 ml of toluene, 1.34 ml of an aqueous solution of 2M Na₂CO₃, then 43.7 mg of Pd-tetrakistriphenylphosphine, and finally 398 mg (1.39 mmol) of 3-(1-adamantyl)-4-methoxyphenylboronic acid were added, and the mixture refluxed for 3 hours. The crude product was taken up in ethyl ether, the organic phase washed with 15 a saturated NaCl solution, dried over Na2SO4, evaporated to dryness to give 570 mg of crude product. Flash chromatography on silica gel (Merck) with hexane:ethyl acetate 2:1 as eluent gave 15 mg of pure product. M.p. 175°C. ¹H-NMR (CDCl₃) δ: 3.86 (s, 3H, OCH3), 3.90 (s, 3H, OCH3), 6.96 (d, 1H, J = 8.5), 7.43 (dd, 1H, J = 2.2, 8.5), 7.47 (d, 1H, J = 2.2), 7.55.7.70 (4H arom.).
B) *4-(3-(1-adamantvl)-4-methoxvphenyl)propiolic acid (7)* 15 mg (0.0374 mmol) of methyl E-4-(3-(1-adamantyl)-4-methoxyphenyl)propiolate were dissolved in 2.14 ml of 0.7N NaOH in methanol, and the mixture refluxed for 1 hour. Methanol was evaporated, the residue taken up in water, acidified with 6N HCl, and extracted with ethyl ether. After drying over Na2S04 and having evaporated the solvent, the residue was washed with hexane, from which after filtration 10 mg of product were obtained M.p. 156°C. Rf= 0.41 (Merck silica gel 6OF254, EtOAc/ MeOH 2/1) ¹H-NMR (DMSO-d₆) δ: 1.70 (s, 6H), 2.10 (s, 9H), 3.85 (s, 3H, OCH3), 7.05 (d, 1H, J =8.4, H-6'), 7.40 (d, 1H, J = 2, H-2'), 7.45-7.60 (3H arom.), 7.65 (2H arom.)

### Example 8: Synthesis of 3-(3'-Adamantan-1-yl-4-hydroxybiphenyl-4-yl)-N-hydroxyacrylamide (8)

To a suspension of 3-(3'-adamantan-1-yl-4'-hydroxy-biphenyl-4-yl)-acrylic acid (0.53 mmol) in dry DMF (8.3 mL) HOBt (86 mg, 0.64 mmol) and WSC (132 mg, 0.69 mmol) were added. The mixture was stirred at room temperature under nitrogen for 3 h, then NH₂OH.HCl (185 mg, 2.66 mmol) and TEA (368 mL, 2.66 mmol) were added. The reaction was stirred at room temperature for 4 h. After addition of water, a precipitate formed, which was filtered and dried to obtain the desired hydroxamic acid. The crude precipitate was purified by flash chromatography with CH3OH/H2O 75:25 v/v on RP-18 silica gel. Yield: 54%. M.p. 184°C. ¹H NMR (DMSO-d6) δ: 10.70 (1H, brs); 9.50 (1H, s); 9.01 (1H,brs); 7.65e7.50 (4H, m); 7.45 (1H, d, J = 16.2 Hz); 7.35e7.25 (2H, m); 6.82 (1H, d, J = 8.8 Hz); 6.45 (1H, d, J = 16.2 Hz); 2.12 (6H, s); 2.04 (3H, s); 1.72 (6H, s). ¹³C NMR (DMSO-d₆) δ: 162.9, 156.4, 141.9, 138.1, 136.0, 132.7, 129.9, 128.1, 126.4, 124.9, 124.7, 118.3, 117.0, 38.7, 36.7, 36.3, 28.4.

### Example 9: Synthesis of (E)-3-(3'-adamantan-1-yl)-4'-(2-(hydroxyamino)-2-oxoethoxy)-[1,1'-biphenyl]-4-yl)acrylic acid (9)

*A) 3-(3'-Adamantan-1-yl-4'-ethoxycarbonylmethoxy-biphenyl-4-yl)-acrylic acid tert-butyl ester.* A mixture of 3-(3'-adamantan-1-yl-4'-hydroxy-biphenyl-4-yl)-acrylic acid tert-butyl ester (2.00 g, 4.64 mmol), ethyl bromoacetate (1.58 g, 9.28 mmol), K₂CO₃ (2.57 g, 18.6 mmol) in 40 ml of DMF was heated at 80 °C for 1 h. K₂CO₃ was filtered, DMF evaporated and the residue was taken up with water. The solid precipitated was filtered and crystallized from diethyl ether to give 2.09 g ( 97 %) of product as white solid, m.p. 175°C, Rf ( petroleum ether/ethyl acetate 90:10) 0.31. ¹H-NMR (300 MHz, CDCl₃) δ: 7.61 (1H, d, J= 15.9 Hz), 7.58-7.53 (4H, m), 7.50 (1H, d, J= 2.35 Hz), 7.39 (1H, dd, J= 8.39 Hz, J= 2.35 Hz), 6.79 (1H, d, J= 8.39 Hz), 6.38 (1H, d, J= 15.9 Hz), 4.67 (2H, s), 4.31 (2H, q, J= 7.16 Hz), 2.23-2.07 (9H, m), 1.88-1.74 (6H, m), 1.54 ( 9H, s), 1.32 (3H, t, J= 7.16 Hz). ¹³C-NMR (75 MHz, CDCl3)δ: 168.7, 166.4, 156.7, 143.2, 142.9, 139.2, 133.2, 132.9, 128.4 (× 2C), 127.1 (× 2C), 126.0, 125.2, 119.6, 112.2, 80.4, 65.4, 61.3, 40.5 (× 3C), 37.2, 37.0 (× 3C), 29.1 (× 3C), 28.2 (× 3C), 14.2.
B) *3-(3'-Adamantan-1-yl-4'-carboxymethoxy-biphenyl-4-yl)-acrylic acid tert-butyl ester.* To a solution of 3-(3'-Adamantan-1-yl-4'-ethoxycarbonylmethoxy-biphenyl-4-yl)-acrylic acid tert-butyl ester (2.08 g, 4.02 mmol) in 125 ml of aq. 50% THF was added with 843 mg (20.1 mmol) of LiOH.H2O and left overnight at rt. Evaporation, taking up with EtOAc, washing with 60 ml of 1N KHSO4, extraction with EtOAc, drying the extracts with Na2SO4, filtration and evaporation gave 1.92 g (98%) of the product, white solid, m.p.> 300°C, Rf (CH₂Cl₂/ MeOH 19 : 1) 0.23. 1H-NMR (300MHz, DMSO-d₆)δ: 7.75-7.60 (4H, m), 7.56 (1H, d, J= 15.8 Hz), 7.48 (1H, dd, J= 8.51 Hz, J= 2.36 Hz), 7.43 (1H, d, J= 2.36 Hz), 6.93 (1H, d, J= 8.51 Hz), 6.51 (1H, d, J= 15.8 Hz), 4.70 (2H, s), 2.19-1.98 (9H, m), 1.83-1.66 (6H, m), 1.48 (9H, s). ¹³C-NMR (75MHz, DMSO-d₆)δ: 170.4, 166.1, 157.2,143.7, 142.5, 138.6, 132.9, 132.1, 129.3 (× 2C), 127.0 (× 2C), 125.5, 125.2, 119.7, 113.5, 80.3, 65.3, some signals are missing due to the overlap with solvent, 37.1, 37.9, 28.9 (× 3C), 28.3 (× 3C).
C) *3-{3'-Adamanan-1-yl-4'-[(tetrahydropyran-2-yloxycarbamoyl)-methoxy]-biphenyl-4-yl}-acrylic acid tert-butyl ester.* Into an ice-cooled suspension of 3-(3'-Adamantan-1-yl-4'-carboxymethoxy-biphenyl-4-yl)-acrylic acid tert-butyl ester (1.25 g, 2.56 mmol) in 26 ml of dry DMF, under nitrogen, HBTU (0.971 g, 2.56 mmol) and DIPEA (0.993 g, 7.68 mmol) were added. After two minutes 0.312 g (2.56 mmol) of O-(tetrahydro-pyran-2-yl)-hydroxylamine were added and the mixture was left overnight at rt. DMF was evaporated, the residue was taken up with water and the white solid filtered. Purification by flash chromatography on silica gel with petroleum ether/EtOAc 7:3 gave 1.23 g of the product, white solid, m.p. 193 °C, Rf (AcOEt/petroleum ether 3:7) 0.31. ¹H-NMR (300 MHz, CDCl3)δ: 9.13 (1H, s), 7.61 (1H, d, J= 16.0 Hz), 7.58-7.54 (4H, m), 7.51 (1H, d, J= 2.24 Hz), 7.43 (1H, dd, J= 8.53 Hz, J= 2.24 Hz), 6.91 (1H, d, J= 8.53 Hz), 6.39 (1H, d, J= 16.0 Hz), 5.07-5.02 (1H, m), 4.68 (2H, s), 4.00-3.90 (1H, m), 3.67-3.57 (1H, m), 2.18-2.11 (9H, m), 1.92-1.78 (8H, m), 1.70-1.58 (4H, m), 1.54 (9H, s). ¹³C-NMR (75 MHz, CDCl3)δ: 166.4, 165.1, 156.1, 143.1, 142.5, 138.8, 134.2, 133.2, 128.4 (× 2C), 127.1 (× 2C), 126.2, 125.6, 119.8, 113.4, 102.8, 80.5, 67.6, 62.5, 41.1 (× 3C), 37.1, 37.0 (× 3C), 28.9 (× 3C), 28.2 (× 3C), 27.9, 24.9, 18.5.
D) *(E) -3-(3'-adamantan-1-yl)-4'-(2-(hydroxyamino)-2-oxoethoxy)-[1,1'-biphenyl]-4-yl)acrylic acid.* 3-{3'-Adamantan-1-yl-4'-[(tetrahydropyran-2-yloxycarbamoyl)-methoxy]-biphenyl-4-yl}-acrylic acid tert-butyl ester (250 mg, 0.425 mmol) was added portionwise to 3.5 ml of TFA at 0°C under nitrogen and the mixture was stirred at 0 °C for 1h. The pink suspension was filtered, the solid was washed with water until neutral pH to give 175 mg (92%) of desired compound as white solid, m.p. 265 °C, Rf (CH₂Cl₂/ MeOH 18:2) 0.49. ¹H-NMR (300 MHz, CDCl₃ +TFA)δ: 7.91 (1H, d, J = 16.0 Hz), 7.67-7.59 (4H, m), 7.55 (1H, d, J = 2.22 Hz), 7.47 (1H, dd, J= 8.30 Hz, J= 2.22 Hz), 6.90 (1H, d, J= 8.30 Hz), 6.49 (1H, d, J= 16.0 Hz), 4.78 (2H, s), 2.20-2.10 (9H, m), 1.91-1.72 (6H, m). ¹³C-NMR (75 MHz, DMSO-d₆)δ: 168.1, 164.8, 157.7, 144.0, 142.5, 138.8, 133.0, 132.3, 129.2 (×2C), 127.1 (×2C), 125.6, 125.2, 119.1, 114.0, 66.4, (5C missing due to the overlap with signal solvent), 37.1, 36.9, 28.9 (×3C).

### Example 10: Synthesis of (E)-3-(3'-adamantan-1-yl)-4'-((1-(hydroxyamino)-1-oxopropan-2-yl)oxy)-[1,1'-bipheny1]-4-yl)acrylic acid (10)

*A) (E)-3-(3'-adamantan-1-yl)-4'-((1-ethoxy-1-oxopropan-2-yl)oxy)-[1,1'-biphenyl]-4-yl)acrylic acid tert-butyl ester* To a solution of 3-(3'-adamantan-1-yl-4'-hydroxy-biphenyl-4-yl)-acrylic acid tert-butyl ester (500 mg, 1.16 mmol) in dry acetone (7 mL), K₂CO₃ (641 mg, 4.64 mmol), KI (13 mg, 0.08 mmol) and 2-bromopropionic acid ethyl ester (180 µL, 1.39 mmol), were added under nitrogen. The mixture was heated 5h to reflux. K₂CO₃ was filtered, and the solvent removed in vacuo. The crude was triturated with hexane: diethyl ether 5:1, the precipitate was filtered to give 340 mg of title compound. Yield: 55%. m.p. 88°C, Rf (petroleum ether/ethyl acetate 92.5:7.5) 0.30.
   ¹H-NMR (300 MHz, CDCl₃)δ : 7.61 (1H, d, J = 15.7 Hz), 7.57-7.52 (4H, m), 7.49 (1H, d, J = 2.3 Hz), 7.35 (1H, dd, J = 2.3, 8.4 Hz), 6.71 (1H, d, J = 8.4 Hz), 6.38 (1H, d, J = 15.7 Hz), 4.88 (1H, q, J = 6.6 Hz), 4.22 (2H, q, J = 7.1 Hz), 2.31-2.14 (6H, m), 2.13-2.04 (3H, m), 1.84-1.74 (6H, m), 1.71 (3H, d, J = 6.6 Hz), 1.54 (9H, s), 1.25 (3H, t, J = 7.1 Hz).
*B) (E)-3-(3'-adamantan-1-yl)-4'-(1-carboxyethoxy)-[1,1'-biphenyl]-4-yl)acrylic acid tert-butyl ester*
   To a solution of (E)-3-(3'-adamantan-1-yl)-4'-((1-ethoxy-1-oxopropan-2-yl)oxy)-[1,1'-biphenyl]-4-yl)acrylic acid tert-butyl ester (500 mg, 0.94 mmol) in aq. 50% THF (30 mL), LiOH.H2O (198 mg, 4.71 mmol) was added. The solution was stirred overnight at rt. The solvent was evaporated, the crude was diluted in 1N KHSO₄ and extracted with EtOAc. The combined organic extracts were washed with brine, dried over anhydrous Na₂SO₄ and concentrated in vacuo, to give 462 mg (98%) of title compound (white solid), m.p. 206°C, R_{f} (CH₂Cl₂/ MeOH 193: 7) 0.23. ¹H-NMR (300MHz, DMSO-*d₆*) δ: 7.76-7.60 (m, 4H), 7.56 (d, 1H, J = 15.9 Hz), 7.47 (dd, 1H, J = 2.0, 8.4 Hz), 7.43 (d, 1H, J = 2.0 Hz), 6.80 (d, 1H, J = 8.4 Hz), 6.51 (d, 1H, J = 15.9 Hz), 4.91 (q, 1H, J = 6.5 Hz), 2.24 - 2.00 (m, 9H), 1.79-1.66 (m, 6H), 1.57 (d, 3H, J = 6.5 Hz), 1.47 (9H, s).
*C) (E)-3-(3'-adamantan-1-yl)-4'-((1-oxo-1-(((tetrahydro-2H-pyran-2-yl)oxy)amino)propan-2-yl)oxy)-[1,1'-biphenyl]-4-yl)acrylic acid tert-butyl ester*
   To a solution of (E)-3-(3'-adamantan-1-yl)-4'-(1-carboxyethoxy)-[1,1'-biphenyl]-4-yl)acrylic acid tert-butyl ester (400 mg, 0.79 mmol) in dry DMF (6 mL), HBTU (302 mg, 0.79 mmol) and DIPEA (423 µL, 2.38 mmol) were added at 0°C under nitrogen atmosphere. The solution was stirred 30 min at 0°C then O-(tetrahydropyran-2-yl)-hydroxylamine (93 mg, 079 mmol) was added and the reaction was stirred overnight at rt. The solvent was removed in vacuo, the residue was triturated with water and filtered. The crude was purified by flash column chromatography in hexane: ethyl acetate 3 : 1 to give 400 mg of title compound. Yield: 84%, Rf (hexane :ethyl acetate 7 : 3) 0.32. ¹H-NMR (300 MHz, CDCl₃) δ: mixture of stereoisomer 8.98 (1H, s), 8.87 (1H, s), 7.61 (2H, d, J = 15.8 Hz), 7.57-7.53 (8H, m), 7.51 (2H, d, J = 2.3 Hz), 7.45-7.35 (2H, m), 6.91-6.83 (2H, m), 6.38 (2H, d, J = 15.9 Hz), 5.03-4.81 (4H, m), 3.90-3.76 (2H, m), 3.64-3.54 (1H, m), 3.50-3.38 (1H, m), 2.26-2.07 (18H, m), 1.84-1.76 (12H, m), 1.69-1.74 (6H, m), 1.54 (18H, s).
D) *(E)-3-(3'-adamantan-1-yl)-4'-((1-(hydroxyamino)-1-oxopropan-2-yl)oxy)-[1,1'-biphenyl]-4-yl)acrylic acid*
   To a solution of *(E)-3-(3'-adaman^{t}an-1-yl)-4'-((1-oxo-1-(((tetrahydro-2H-pyran-2-yl)oxy)amino)propan-2-yl)oxy)-[1,1'-biphenyl]-4-yl)acrylic acid tert-butyl ester* (1.35 g, 2.20 mmol) in dry CH₂Cl₂ (22 mL) at 0°C, TFA (22 mL) was dropwise under nitrogen. The solution was stirred at 0 °C for 4h. The mixture was evaporated and treated with toluene to remove TFA, the solid obtained was triturated in CH₂Cl₂ and filtrated to give 646 mg of title compound. Yield: 64%., Rf (CH₂Cl₂/ MeOH/ H₂O 18: 2 : 0.2) 0.35. ¹H-NMR (300 MHz, CDCl₃+TFA) δ: 7.92 (1H, d, J = 15.9 Hz), 7.68-7.59 (4H, m), 7.57 (1H, d, J = 2.2 Hz), 7.44 (1H, dd, J = 2.2 Hz, 8.3 Hz), 6.80 (1H, d, J = 8.3 Hz), 6.49 (1H, d, J = 15.9 Hz), 5.10-4.95 (1H, m), 2.28-2.03 (9H, m), 1.91-1.68 (9H, m).

### Example 11: Synthesis of 3-[3'-Adamantan-1-yl-4'-(hydroxy-butoxy)-biphenyl-4-yl]-acrylic acid (11)

A) *3-[4'-(4-Acetoxy-butoxy)-3'-adamantan-1-yl-biphenyl-4-yl]-acrylic acid methyl ester* A mixture of 3-(3'-adamantan-1-yl-4'-hydroxy-biphenyl-4-yl)-acrylic acid methyl ester (100 mg, 0.26 mmol), 4-bromobutyl acetate (67.4 mg, 0.34 mmol), K₂CO₃ (102 mg, 0.74 mmol) in DMF (4.50 mL) was heated 2h at 80 °C. Then, K₂CO₃ was filtered, DMF evaporated and the residue was diluted with ethyl acetate and washed with 1M HCl, water and brine. The organic phase was dried over anhydrous Na₂SO₄ and the solvent evaporated. Purification by flash chromatography (petroleum ether/ethyl acetate 80: 20) gave 98 mg (75 %) of product as white solid, m.p. 157 °C, R*_{f}* (petroleum ether/EtOAc 80:20) 0.50. ¹H-NMR (300 MHz, DMSO-*d₆*) δ: 7.76-7.60 (4H, m); 7.56 (1H, d, J =16.5 Hz); 7.48 (1H, dd, J = 8.50, 2.14 Hz); 7.43 (1H, d, J = 2.14 Hz); 6.95 (1H, d, J = 8.50 Hz); 6.51 (1H, d, J = 16.5 Hz); 4.84 (2H, s); 3.73 (3H, s); 4H missing due to the overlap with signal solvent; 2.20-1.98 (9H, m); 1.84-1.65 (6H, m); 1.47 (7H, s).
B) *3-[3'-Adamantan-1-yl-4'-(hydroxy-butoxy)-biphenyl-4-yl]-acrylic acid.* A stirred suspension of 3-[4'-(4-Acetoxy-butoxy)-3'-adamantan-1-yl-biphenyl-4-yl]-acrylic acid methyl ester (30 mg, 0.06 mmol) in 0.7 N NaOH in was refluxed for 10 h. After removal of methanol, the residue was treated with cold water, acidified with 1M HCl, and the precipitate was filtered. Crystallization from isopropyl ether gave 10 mg (37%) of the pure product as white solid, m.p. 208°C, R*_{f}* (petroleum ether/EtOAc 50:50) 0.15. ¹H-NMR (300 MHz, DMSO-*d₆*) δ: 7.74-7.61 (4H, m); 7.59 (1H, d, *J* = 16.5 Hz); 7.49 (1H, dd, *J* = 8.80, 2.11 Hz); 7.41 (1H, d, *J* = 2.11 Hz); 7.03 (1H, d, *J* = 8.80 Hz); 6.51 (1H, d, *J* = 16.5 Hz); 4.49 (1H, brs); 4.02 (2H, t, *J* = 6.38 Hz); 3.48 (2H, t, *J* = 6.38 Hz); 2.16-2.20 (9H, m); 1.90-1.78 (2H, m); 1.77-1.59 (8H, m).

### Example 12: Synthesis of 3-[3'-Adamantan-1-yl-4'hydroxy-2-tert-butoxyiminomethyl-biphenyl-4-yl]-acrylic acid (12)

A) Methyl 3-[3'-adamantan-1-yl-4'-(tert-butyl-dimethyl-silanyloxy)-2-tert-butoxyiminomethyl-biphenyl-4-yl]-acrylate. A solution of 100 mg (0.19 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry, 2005, 48, (4931-4946) in 9 ml of ethanol is added with 1.4 ml of pyridine and 78 mg (0.62 mmol) of O-tertbutylhydroxylamine hydrochloride, and refluxed for 1 hour. Evaporation gave a crude that was used for the next step without further purification.
B) 3-[3'-Adamantan-1-yl-4'-hydroxy-2-tert-butoxyiminomethyl-biphenyl-4-yl]-acrylic acid. 100 mg (0.18 mmol) of methyl 3-[3'-adamantan-1-yl-4'-(tert-butyl-dimethyl-silanyloxy)-2-tert-butoxyiminomethyl-biphenyl-4-yl]-acrylate are dissolved in 6 ml of a solution 1N of NaOH in methanol and the mixture refluxed for 5 hours. Evaporation, taking up with water, cooling with ice, slow addition of HCl 1N to acid pH, filtration, and drying gives 65 mg of a yellowish product, m.p. 239°C. ¹H-NMR (300 MHz, CDCl₃) δ: 8.12 (1H, s); 8.06 (1H, s); 7.87 (1H, d, J = 16.2 Hz); 7.59 (1H, dd, J = 8.2, 1.5 Hz); 7.38 (1H, d, J = 8.2 Hz); 7.18 (1H, d, J = 1.8 Hz); 7.05 (1H, dd, J = 8.2, 1.8 Hz); 6.73 (1H, d, J = 8.2 Hz); 6.54 (1H, d, J = 16.2 Hz); 2.19-2.05 (9H, m), 1.80 (6H, s); 1.45 (9H, s).

### Example 13: Synthesis of 3-[3'-Adamantan-1-yl-4'-hydroxy-2-methoxyimino-methyl-biphenyl-4-yl]-acrylic acid (13)

A) Methyl 3-[3'-adamantan-1-yl-4'-(tert-butyl-dimethyl-silanyloxy)-2-methoxyiminomethyl-biphenyl-4-yl]-acrylate. A solution of 100 mg (0.19 mmol) of methyl 3-[3'-adamantan-1-yl-4'-(tert-butyl-dimethyl-silanyloxy)-2-formyl-biphenyl-4-yl]-acrylate (R. Cincinelli et al. Journal of Medicinal Chemistry (2005) ,48,4931-4946) in 9 ml of ethanol is added with 1.4 ml of pyridine and 52 mg (0.62 mmol) of O-methylhydroxylamine hydrochloride, and refluxed 1 hour. Evaporation gave a crude that was used for the next step without further purification.
B) 3-[3'-Adamantan-1-yl-4'-hydroxy-2-methoxyimino-methylbiphenyl-4-yl]-acrylic acid
   85 mg (0.15 mmol) of methyl 3-[3'-adamantan-1-yl-4'-(tert-butyldimethyl-silanyloxy)-2-methoxyimino-methyl-biphenyl-4-yl]-acrylate are dissolved in 5 ml of a solution 1N of NaOH in methanol and the mixture refluxed for 5 hours. Evaporation, taking up with water, cooling with ice, slow addition of HCl 1N to acid pH, filtration, and drying give a yellowish product, 55 mg, m.p. 224°C. ¹H-NMR (300 MHz, CDCl₃) δ: 8.13 (1H, s); 8.07 (1H, s); 7.85 (1H, d, J = 16.2 Hz); 7.60 (1H, dd, J = 8.2, 1.8 Hz); 7.40 (1H, d, J = 8.2 Hz); 7.18 (1H, d, J = 2.1 Hz); 7.03 (1H, dd, J = 8.2, 2.1 Hz) 6.74 (1H, d, J = 8.2 Hz); 6.55 (1H, d, J = 16.2 Hz); 4.00 (3H, s); 2.21-2.06 (9H, m), 1.80 (6H, s).

### Example 14: Synthesis of 3-[3'-Adamant-1-yl-4'-hydroxy-2-carboxymethoxyiminomethyl-biphenyl-4-yl]-acrylic acid. (14)

A solution of 70 mg of methyl 3-[3'-adamantan-1-yl-4'-hydroxy-2-(formyl)-biphenyl-4-yl]-acrylic acid in 4 ml of ethanol is treated with 1 eq of pyridine, then with 20 mg of aminoxyacetic acid and left at room T for 24 hrs. Evaporation and column chromatography with CH₂Cl₂:MeOH 4:1 give 65 mg of the product, m.p. 108-110°C (dec). ¹H-NMR (300 MHz, DMSO-*d₆*) δ: 9.68 (1H,s); 8.07 (1H, s); 7.93 (1H, d, J= 1.53 Hz); 7.82 (1H, dd, J= 8.24 Hz, 1.53 Hz); 7.64 (1H, d, J= 16.2 Hz); 7.43 (1H, d, J= 8.24 Hz); 7.06-6.99 (2H, m); 6.88 (1H, d, J= 8.24 Hz); 6.54 (1H, d, J= 16.2 Hz); 4.66 (2H, s); 2.14-1.99 (9H, m); 1.72 (6H, s).

### Example 15: Synthesis of 3-(3'-Adamantan-1-yl-2-guanidinomethyl-4'-hydroxy-biphenyl-4-yl)-acrylic acid trifluoroacetate (15).

### A) 3-(3'-Adamantan-1-yl-2-(ditertbutoxycarbonylguanidino)methyl)-4'-hydroxy-biphenyl-4-yl)-acrylic acid

A suspension of 113 mg (0.22 mmol) of 3-(3'-adamantan-1-yl-2-aminomethyl-4'-hydroxy-biphenyl-4-yl)-acrylic acid trifluoroacetate in 2.2 ml of dichloromethane is added with 60 µl (2 eq.) of triethylamine, then, after 5 minutes, added with 94 mg (0.24 mmol) of N,N'-Di-Boc-N"-trifluoromethanesulfonyl-guanidine and another eq. of triethylamine, and left under a nitrogen atmosphere and in the dark for 12 hours. Taking up with 15 ml of dichloromethane, washing with a solution 2M of NaHSO₄, washing with water, drying over Na₂SO₄, and evaporation gives 133 mg of a crude product. Crystallization from dichloromethane/methanol (5 ml : 0.25 ml) gives 30 mg of pure product, whereas chromatography of the mother liquid with dichloromethane/methanol gives further 70 mg, m.p. 280°C. ¹H-NMR (300 MHz, MeOD) δ: 7.73-7.64 (m, 2H), 7.58 (dd, J = 8.2, 1.8 Hz, 1H), 7.31 (d, J = 8.2 Hz, 1H), 7.04 (d, J = 2.1 Hz, 1H), 6.96 (dd, J = 8.2, 2.1 Hz, 1H), 6.76 (d, J = 8.2 Hz, 1H), 6.50 (d, J = 16.2 Hz, 1H), 4.31 (s, 2H), 2.15-1.94 (m, 9H), 1.77 (s, 6H), 1.46 (s, 9H), 1.42 (s, 9H).

B) *3-(3'-Adamantan-1-yl-2-guanidinomethyl-4'-hydroxy-biphenyl-4-yl)-acrylic acid trifluoroacetate.* A solution of 70 mg (0.11 mmol) of 3-(3'-adamantan-1-yl-2-(ditertbutoxycarbonylguanidino)methyl)-4'-hydroxy-biphenyl-4-yl)-acrylic acid in 1.1 ml of dichloromethane is slowly added under nitrogen atmosphere, 1.1. ml of trifluoroacetic acid, and left 12 hours in the dark. Evaporation and chromatography with silica gel with CH₂Cl₂/MeOH/TFA 18:2:0.2 as an eluent gives a hygroscopic product, (67 mg, m.p. 163°C). ¹H-NMR (300 MHz, MeOD) δ: 7.69 (d, J = 16.2 Hz, 1H), 7.64-7.58 (m, 2H), 7.32 (d, J = 8.2 Hz, 1H), 7.08 (d, J = 1.5 Hz, 1H), 6.97 (dd, J = 8.2, 1.5 Hz, 1H), 6.78 (d, J = 8.2 Hz, 1H), 6.50 (d, J = 16.2 Hz, 1H), 4.27 (s, 2H), 2.20-1.96 (m, 9H), 1.78 (s, 6H).

### Example 16: Synthesis of 3-{4-[8-(Adamantan-1-yl)-2,3-dihydro-benzo[1,4]-dioxin-6-yl]-phenyl}-acrylic acid (16).

3-{4-[8-(Adamantan-1-yl)-2,3-dihydro-benzo[1,4]-dioxin-6-yl]-phenyl}-acrylic acid (16). Suzuki one-pot condensation between 200 mg (0.573 mmol) of 4-bromo-1,2-ethylenedioxybenzene with methyl 4-bromocinnamate was carried out as described in R. Cincinelli et al. / Bioorg. Med. Chem. 15 (2007) 4863-4875. The residue was purified by flash chromatography (dichloromethane/ hexane 1.1) to give 124 mg (50%) of 3-{4-[8-(adamantan-1-yl)-2,3-dihydro-benzo[1,4]dioxin-6-yl]-phenyl}- acrylic acid methyl ester, mp 209 °C. ¹H NMR (300 MHz, CDCl₃) δ: 1.78 (6H, s, 6Ad), 2.08 (3H, s, 3Ad), 2.13 (6H, s, 6Ad), 3.83 (3H, s, OMe), 4.33 (4H, s, OCH2), 6.45 (1H, d, CH=, J = 16.0 Hz), 7.00 (1H, d, 1Ar, J = 2.3 Hz), 7.05 (1H, d, 1Ar, J = 2.3 Hz), 7.50-7.63 (4H, m, 4Ar), 7.70 (1H, d, CH=, J = 16.0 Hz). The above ester (70 mg, 0.163 mmol) was hydrolysed with LiOH to afford 62 mg (91%) of the pure product, mp 288 °C. ¹H NMR (300 MHz, DMSO-d6) δ: 1.71 (6H, s, 6Ad), 2.02 (3H, s, 3Ad), 2.08 (6H, s, 6Ad), 4.25 (4H, s, OCH2), 6.50 (1H, d, CH@, J = 15.6 Hz), 6.97 (1H, d, 1Ar, J = 1.9 Hz), 7.02 (1H, d, 1Ar, J = 1.9 Hz), 7.56 (1H, d, CH@, J = 15.6 Hz), 7.58 (2H, d, 2Ar, J = 8.25 Hz), 7.69 (2H, d, 2Ar, J = 8.3 Hz).

### Example 17: Synthesis of E-3-[3'-(Acetylaminomethyl)-4'-hydroxy-5'-(adamantan-1-yl)biphenyl-4-yl]-acrylic acid (17).

A) N-[5-Bromo-2-hydroxy-3-(adamantan-1-yl)-benzyl] acetamide. A finely pulverized mixture of 2-(1-adamantanyl)-4-bromophenol (500 mg, 163 mmol) and N-(hydroxymethyl)acetamide (145 mg, 1.63 mmol) was added portionwise, at 10 °C while stirring, to 1.63 mL of a solution of CH₃COOH/H₂SO₄ 9:1. After the mixture was stirred at room temperature for 5 days, ice was added and the yellow solid was filtered and washed with water. Purification by flash chromatography (hexane/ethyl acetate 60:40) gave 225 mg (37%) of the pure product, mp 216 °C ¹H NMR (300 MHz, CDCl₃) d: 1.77 (6H, s, 6Ad), 2.04 (3H, s, 3Ad), 2.05(3H, s, CH3), 2.10 (6H, s, 6Ad), 4.25 (2H, d, CH2N, J = 6.8 Hz), 6.28 (1H, t, NH, J = 6.8 Hz), 7.05 (1H, d, 1Ar, J = 2.6 Hz), 7.23 (1H, d, 1Ar, J = 2.6 Hz). 6.4.10.
B) E-3-[3'-(Acetylaminomethyl)-4'-hydroxy-5'-(adamantan-1-yl)biphenyl-4-yl]-acrylic acid (17). An amount of 195 mg (0.516 mmol) of N-[5-Bromo-2-hydroxy-3-(adamantan-1-yl)-benzyl] acetamide was reacted with methyl p-bromocinnamate according to the general procedure described in R. Cincinelli et al. / Bioorg. Med. Chem. 15 (2007) 4863-4875. Purification by flash chromatography (ethyl acetate/dichloromethane 9:1) afforded 103 mg (43%) of 3-[3'-(acetylaminomethyl)-4'-hydroxy-5'-(adamantan-1-yl)biphenyl- 4-yl]-acrylic acid methyl ester, mp 163 °C. ¹H NMR (300 MHz, CDCl₃) δ: 1.80 (6H, s, 6Ad), 2.05(3H, s, OAc), 2.08 (3H, s, 3Ad), 2.20 (6H, s, 6Ad),3.80 (3H, s, OMe), 4.40 (2H, d, CH2N, J = 6.0 Hz),6.32 (1H, t, NH, J = 6.0 Hz), 6.69 (1H, d, CH=,J = 15.8 Hz), 7.19 (1H, d, 1Ar, J = 2.6 Hz), 7.44 (1H, d, 1Ar, J = 2.6 Hz), 7.54-7.58 (4H, m, 4Ar), 7.72 (1H,d, CH=, J = 15.8 Hz). The above ester (100 mg, 0.218 mmol) was hydrolyzed as described in the general procedure described in R. Cincinelli et al. / Bioorg. Med. Chem. 15 (2007) 4863-4875, to obtain 70 mg (72%) of the pure product, mp 264 °C. ¹H NMR (300 MHz, acetone-d₆) δ: 1.83 (6H, s, 6Ad), 2.10 (3H, s, Ac), 2.07 (3H, s, 3Ad), 2.25 (6H, s, 6Ad), 4.36 (2H, d, CH2N, J = 6.0 Hz), 6.53 (1H, d, CH=, J = 15.8 Hz), 7.40 (1H, d, 1Ar, J = 2.3 Hz), 7.48 (1H, d, 1Ar, J = 2.3 Hz), 7.64 (2H, d, 2Ar, J = 8.3 Hz), 7.69 (1H, d, CH=, J = 15.8 Hz), 7.73 (2H, d, 2Ar, J = 8.3 Hz), 8.37 (1H, t, NH, J = 6.0 Hz), 10.41 (1H, s).

### Example 18: Synthesis of (E)-3-(3'-adamantan-1-yl)-4'-hydroxy-5'-((3-phenylureido)methyl)-[1,1'-biphenyl]-4-yl)acrylic acid (18)

To a suspension of 33 mg of E-3-{3'-[(2-Chloroacetylamino)-methyl]-4'-hydroxy-5'-(adamantan-1-yl)biphenyl-4-yl}-acrylic acid (R. Cincinelli et al. / Bioorg. Med. Chem. 15 (2007) 4863-4875) in TEA (4 ml), DMF (2 ml) and DMSO (0.2 ml) were added, followed by phenylisocyanate (30 µl). The mixture was stirred 5 days at room temperature. The solvent was evaporated and water (2 ml) was added, followed by 2N HCl (100 µl). The yellow solid formed was filtered and dried (Yield 80%). ¹H NMR (300 MHz, DMSO-d₆) δ: 1.80 (6H, s, 6Ad), 2.07 (3H, s, 3Ad), 2.21 (6H, s, 6Ad), 4.31 (2H, d, CH2N, J = 6.0 Hz), 6.50 (1H, d, CH=, J = 16.0 Hz), 6.90-7.80 (12H, m), 8.05 (1H, m), 8.90 (1H, s), 10.05 (1H, s).

### Example 19: Synthesis of 3-(3'-Adamantan-1-yl-4'-hydroxybiphenyl-4-yl)propionic Acid (19).

3-(3'-Adamantan-1-yl-4'-hydroxybiphenyl-4-yl)propionic Acid (19). Methyl E-3-(3'-Adamantan-1-yl-4'-hydroxybiphenyl-4-yl)acrylate (80 mg) was dissolved in 40 mL of AcOEt, an amount of 30 mg of PtO₂ was added, and the mixture was hydrogenated for 40 min. After filtration of the catalyst, the solvent was evaporated to obtain 80 mg (100%) of methyl 3-(3'-adamantan-1-yl-4¢-hydroxybiphenyl-4-yl)propionate, mp 150 °C. A solution of 43 mg (1.03 mmol) of LiOH.H2O and 80 mg (0.205 mmol) of the above ester in 8.4 mL of THF/H₂O, 1:1, was stirred at room temperature for 1.5 h. THF was evaporated, and the remaining aqueous layer was washed with hexane and then acidified with 1M HCl to pH 2 to obtain, after filtration, 57 mg of a white solid. Yield 74%, mp 197-200 °C. ¹H NMR (acetone-d6) δ: 1.80 (6H, s, 6Ad), 2.10 (3H, s, 3Ad), 2.25 (6H, s, 6Ad), 2.65 (2H, t, -CH2-, J = 7.72 Hz), 2.95(2H, t, -CH2-, J = 7.72 Hz), 6.90 (1H, d, 1Ar, J = 8.09 Hz), 7.25-7.35 (3H, m, 3Ar), 7.42 (1H, d, 1 Ar, J = 2.21 Hz), 7.50 (2H, d, 2Ar, J = 8.34 Hz), 8.45 (1H, brs, OH).

### Example 20: Synthesis of (E)-Octadeca-9,12-dienoic acid 3-adamantan-1-yl-40-(2-carboxyvinyl)-biphenyl-4-yl ester (20)

To a solution of (E)-3-(30-Adamantan-1-yl-40-hydroxybiphenyl-4-yl)acrylic acid tert-butyl ester (200 mg, 0.464 mmol) and a few crystals of DMAP in 2.19 mL of dry pyridine were added 208 mg (0.696 mmol) of linoleoyl chloride. After stirring overnight at room temperature the reaction mixture was poured into iced water and the aqueous layer was extracted twice with ethyl acetate. The combined organic layers were washed twice with 1 N HCl, water and dried over Na₂SO₄. Flash chromatography of the reaction mixture (hexane/ ethyl acetate 95:5) gave 210 mg of (E)-octadeca-9,12-dienoic acid 3-adamantan-1-yl-40-(2-tert-butoxycarbonylvinyl)-biphenyl-4-yl ester as a colourless oil. Yield 65%, ¹H-MNMR (300 MHz, DMSO-d₆) δ 7.81-7.65 (m, 4H), 7.64-7.53 (m, 3H), 7.10 (d, J = 8.5 Hz, 1H), 6.56 (d, J = 16.0 Hz, 1H), 5.43-5.24 (m, 4H), 2.73 (t, J = 2.25 Hz, 2H), 2.67 (t, J = 3.75 Hz, 2H), 2.10-1.93 (m, 13H), 1.80-1.60 (m, 8H), 1.50 (s, 9H), 1.43-1.21 (m, 14H), 0.85 (t, J = 3.0 Hz, 3H).

(E)-Octadeca-9,12-dienoic acid 3-adamantan-1-yl-40-(2-tertbutoxycarbonylvinyl)-biphenyl-4-yl ester (110 mg, 0.159 mmol) was dissolved in dry methylene chloride (1.59 mL) and treated with TFA (1.59 mL) at 0 °C under stirring. After 30 min at 0 °C the solvent was evaporated to give 100 mg of title compound as a white solid. Yield 99%. ¹H NMR (300 MHz, DMSO-d6) δ 7.84-7.68 (m, 4H), 7.62 (d, J = 15.0 Hz, 1H), 7.58-7.50 (m, 2H), 7.10 (d, J = 8.5 Hz, 1H), 6.58 (d, J = 16.0 Hz, 1H), 5.40-5.22 (m, 4H), 2.76 (t, J = 2.25 Hz, 2H), 2.70 (t, J = 2.65 Hz, 2H), 2.10-1.90 (m, 13H), 1.80-1.67 (m, 8H), 1.45-1.16 (m, 14H), 0.86 (t, J = 3.0 Hz, 3H).

### Example 21: Synthesis of E-3-[3'-Formyl-4'-hydroxy-5'-(adamantan-1-yl)-biphenyl-4-yl]-acrylic acid (21).

A)4'-Bromo-4-hydroxy-5-(adamantan-1-yl)biphenyl-3-carbaldehyde. To a solution of 2 g (5.22 mmol) of 4-(4'-bromophenyl)-2-(adamantan-1-yl)-phenol and 1.12 g (1.22 mL, 10.44 mmol) of 2,6-lutidine in 11 mL of freshly distilled toluene, 0.68 g (2.61 mmol, 0.3 mL) of SnCl₄ was added over 10 min under a nitrogen atmosphere. A pale yellow precipitate was formed. The mixture was allowed to stir at room temperature for 20 min, then solid paraformaldehyde (0.626 g, 20.88 mmol) was added in one portion and the reaction mixture was stirred for additional 10 min, then heated at 90-95 °C for 7 h. After having cooled at room temperature, 30 mL of water and 6 mL of HCl 1 M were added. The aqueous phase was extracted with ethyl acetate, the extract washed with brine, dried over Na₂SO₄, and evaporated. The crude product was purified by flash chromatography (hexane/ethyl acetate 95:5) to give 1.3 g (60%) of the title compound, mp 189 °C. ¹H NMR (300 MHz, CDCl₃) d: 1.8 (6H, s, Ad), 1.65 (3H, s, Ad), 2.19 (6H, s, Ad), 7.40 (2H, d, 2Ar, J = 8.3 Hz), 7.54 (2H, d, 2Ar, J = 8.3 Hz), 7.56 (1H, d, 1Ar, J = 2.3 Hz), 7.64 (1H, d, 1Ar, J = 2.3 Hz), 9.94 (1H, s, OH), 11.8 (1H, s, CHO).

B) E-3-[3'-Formyl-4'-hydroxy-5'-(adamantan-1-yl)-biphenyl-4-yl]-acrylic acid (21). 4'-Bromo-4-hydroxy-5-(adamantan-1-yl)biphenyl-3-carbaldehyde was subjected to Heck condensation as described in general procedure reported in R. Cincinelli et al. / Bioorg. Med. Chem. 15 (2007) 4863-4875. The pure 3-(3'-formyl-4'-hydroxy-5'-(adamantan-1-yl)biphenyl-4-yl)-acrylic acid methyl ester was obtained after purification by flash chromatography (hexane/ethyl acetate 3:7) and crystallization from diisopropylether. Yield 66%, mp 245 °C. ¹H NMR (300 MHz, CDCl₃) d: 1.56 (6H, s, Ad), 2.07 (3H, s, Ad), 2.20 (6H, s, Ad), 3.58 (3H, s, OMe), 6.46 (1H, d, CH=, J = 15.0 Hz), 7.59 (m, 4H, 4Ar), 7.60 (1H, d, 1Ar, J = 2.3 Hz), 7.70 (1H, d, 1Ar, J = 2.3 Hz), 7.73 (1H, d, CH=, J = 15.0 Hz), 9.95 (1H, s, OH), 11.9 (1H, s, CHO). The above ester was hydrolysed as described in general procedure reported in R. Cincinelli et al. / Bioorg. Med. Chem. 15 (2007) 4863-4875 to obtain the corresponding acid, yield 83%, mp > 300 °C. ¹H NMR (300 MHz, DMSO-d₆) δ: 1.54 (6H, s, Ad), 2.08 (3H, s, Ad), 2.16 (6H, s, Ad), 6.55 (1H, d, CH=, J = 16.0 Hz), 7.59 (1H, d, CH=, J = 16.0 Hz), 7.66-8.18 (5H, m, 5Ar), 8.03 (1H, d, 1Ar, J = 2.3 Hz), 10.05 (1H, s, CHO), 12.05 (1H, s, OH).

### Example 22: Antimicrobial activity

Determination of Minimal Inhibitory Concentration (MIC), Minimal Bactericidal Concentration (MBC), bactericidal curves and killing rate on Gram positive and Gram negative bacteria strains, for the evaluation of the bactericidal activity of compounds of example 1 (n. 1 to 14).

Sensitivity tests have been performed in broth-dilution (micromethod) with the purpose to test the possible bacteriostatic and/or bactericidal power in different compounds, in accordance with CLSI guidelines (Clinical and Laboratory Standards Institute). In details, phenotypic methods based on *in vitro* MIC and MBC determination have been performed on previously isolated bacterial strains, identified and characterized at the level of their antibiotic susceptibility profile. According to MIC and MBC data, the Killing Rate was calculated as the ratio between MBC and MIC on which basis it is possible to define the compounds tested as bactericides (killing rate = 1 -4), or bacteriostatic (killing rate > 4).

Preparation of the solution containing molecules: for each molecule to be tested, solutions were made as DMSO Stock, with a concentration of 2.560 mg/ml, used to test 12 dilutions with the following scalar drug concentrations: 512-256-128-64-32-16-8-4-2-1-0.5-0.25 µg/ml. The scalar dilutions were prepared directly on plates by using Mueller Hinton Broth (MHB) as a solvent.

Bacterial suspension preparation: cryostrains preserved at -80°C were recovered by incubation in growth medium (Brain Heart Infusion Broth, BHI) for 24-48 hours at 37°C followed by passages in agar growth medium (Columbia Blood Agar). Colonies with less than 30 hours are suspended in MHB with the purpose to prepare a bacterial suspension with a turbidity of 0.5 MF, containing approximately 1.5 x 10⁸cfu/ml. This injection is additionally diluted in MHB until reaching a final concentration of 5 x 10⁵ cfu/ml (range 3-7 x 10⁵ cfu/ml). Identical volumes of this bacterial suspension are added on a plate, for each well containing drug scalar dilutions, to obtain a final concentration between 512 a 0.25 µg/ml. The set up of bacterial suspensions as well as the passage on slate has always been performed within 30 minutes, in order not to alter the concentration of the bacterial suspension. Simultaneously, check in broth-microdilution only with solvent (in order to exclude that the effect of growth inhibition might be due to the drug solution solvent), positive growth control in absence of DMSO and drug, and negative purity control only with injection broth are performed. As a consequence the slate is incubated at 37°C for 24 and 48 hours in aerobes. The 24 hours MIC is considered as the minimum drug concentration able to inhibit bacterial growth, which makes it possible to observe the absence of bacterial growth and the absence of turbidity. The same observations are valid to evaluate 48 hours MIC. After the incubation, an equal volume of bacterial suspension from each well is plated on agar growth medium and incubated for 24-48 hours at a temperature of 37°C in aerobes, in order to define the MBC after the incubation. The MBC is evaluated as the minimum concentration of drug able to kill the 99,9% of the initial bacterial population.

Time-killing curves: evaluation of the number of survivors after 2, 6 and 24 hours drug exposition. The bacterial culture at an exponential growth phase is diluted in fresh broth until reaching a concentration between 10⁶ and 10⁷ cfu/ml. Starting from this suspension, the control in drug absence and the solution containing the drug at a concentration of four times the MIC are set up. In order to evaluate the initial bacterial charge, after a sampling at 0 time, other samplings are performed to count the survivors at the end of 2, 6 and 24 incubation hours.

The dilution of bacterial suspension is realized from samplings, in order to make the bacterial count possible.

Bacterial strains: the bactericidal and/or bacteriostatic power of the synthesized compounds can be determined by using standard reference strains such as:
1*. S. Aureus* ATCC 25923 res kan, tob, res streptogramine (sga-sgb, efflux), res mupirocina (at low level), res trimetoprim;
2*. S. Aureus strain 1* (collection) mec a +, res kan tob gen, res quinolones, lincosamides, res streptogramine (sga-sgb), mlsb+sa constituent, mlsb constituent, res mupirocine (low level), res trimetoprim ;
3*. S. Aureus strain 2* (collection) acquired penicillinase, res kan tob, res streptogramine (sga-sgb, efflux), res mupirocine (low level), res trimetoprim;
4*. Enterococcus faecalis* ATCC 51299 (str + gen res at high level, mlsb res, simil van b res);
5*. Enterococcus faecalis strain 1* (collection) (str+ kan res at high level str res at high level tetracycline res);
6*. Enterococcus faecalis strain 2* (collection) (kan res at high level);
7*.E. coli* of Collection: RESISTANT to ampicilline, amox +ac clav, ampicillinr + sulbactam, cefalexine, cefuroxime, cefuroxime axetil, cefixime, cefpodoxime, cefotaxime, ceftazidime, ceftriaxone, netilmicine, tobramicine, ciprofloxcine, trim/sulfametox, esbl pos, SENSITIVE to meropenem and gentamicine, INTERMEDIATE to amikacine;
8. 8. *A. baumannii* of collection: RESISTANT to cefotaxime, ertapenem, imipenem, meropenem, gentamicine, ciprofloxacine, fosfomicine, nitrofurantoin, trim/sulf; SENSITIVE to colistina, INTERMEDIATE to amikacine; 9. P. Aeruginosa of collection: RESISTANT to amox + ac clav, cefotaxime, ertapenem, tigecicline, fosfomicine, trimetoprim/slf SENSITIVE to piperacillina/tazobactam, ceftazidime, cefepime, imipenem, meropenem, amikacine, gentamicine, ciprofloxacine, colistine.

The results of antibacterial activity are reported in the following table 1.

**Table 1**

| | | **S. AUREUS ATCC 25923** | **S. AUREU S strain 1** | **S. AUREU S strain 2** | **E. FAECAL IS ATCC 61299** | **FAECALI S strain 1** | **E. FAECALIS strain 2** | **E. COLI** | **A. BAUMA NNII** | **P. AERUGINOSA** |
|---|---|---|---|---|---|---|---|---|---|---|
| **adarot ene** | **MIC (µg/ml )** | **4-8*** | **4-8*** | **4** | **2** | **2-4*** | **0.5-1** * | **256** | **128** | **256** |
| | MBC (µg/ml ) | 16 | 16 | 8 | 32 | 32 | 16 | / | 512 | / |
| **1** | **MIC (µg/ml )** | | **64-128 *** | | | | **32** | | | |
| | **MBC** (µg/ml ) | | 128 | | | | 128 | | | |
| **2** | **MIC (µg/ml** ) | | **0.5** | | | | **0.5** | | | |
| | MBC (µg/ml ) | | 4 | | | | 2-4 | | | |
| **3** | **MIC (µg/ml )** | | **2** | | | | **0.5-1 *** | | | |
| | MBC (µg/ml ) | | 4-8 | | | | 8 | | | |
| **4** | **MIC (µg/ml )** | | **2** | | | | **0.5-1 *** | | | |
| | MBC (µg/ml ) | | 4 | | | | 4 | | | |
| **5** | **MIC (µg/ml )** | | **2-4 *** | | | | **4** | | | |
| | MBC (µg/ml) | | 8-16 | | | | 16 | | | |
| **6** | **MIC (µg/ml )** | | 64 | | | | | | | |
| **7** | **MIC (µg/ml )** | | 16 | | | | | | | |
| **8** | **MIC (µg/ml )** | | 64 | | | | | | | |
| **9** | **MIC (µg/ml )** | | 128 | | | | | | | |
| **10** | **MIC (µg/ml )** | | 32 | | | | | | | |
| **11** | **MIC (µg/ml )** | **128-256** * | **64-256 *** | **128-256 *** | **64** | **128** | **128** | | | |
| | MBC (µg/ml ) | / | / | / | / | / | / | | | |
| **12** | **MIC (µg/ml )** | **2** | **2-4*** | **2** | **2** | **2** | **2** | **256** | **128** | **256** |
| | MBC (µg/ml ) | 4 | 4 | 2 | 8 | 8 | 4 | / | / | / |
| **13** | **MIC (µg/ml )** | **2** | **2-4 *** | **4** | **8** | **4-8 *** | **4-8 *** | **512** | **128** | **256** |
| | MBC (ug/ml ) | 4 | 4 | 32 | 32 | 16 | 16 | / | 512 | / |
| **14** | **MIC (µg/ml )** | | **64-128** * | | | | **64-128** * | | | |
| | MBC (µg/ml ) | | / | | | | / | | | |
| **15** | **MIC (µg/ml )** | | **16-32** * | | | | **16-32** * | | | |
| | MBC (µg/ml ) | | 32-64 | | | | 32-64 | | | |
| **16** | **MIC (µg/ml )** | | **2 - 4*** | | | | **1 -2 *** | | | |
| **17** | **MIC (µg/ml )** | | **4 -8*** | | | | **2 -4** * | | | |
| | MBC (µg/ml ) | | 16 | | | | 16 | | | |
| **18** | **MIC (µg/ml )** | | **4 -8*** | | | | **2 -4 *** | | | |
| | **MBC** (µg/ml ) | | 8 | | | | 16 | | | |
| **19** | **MIC (µg/ml )** | | **8 - 16 *** | | | | **8 - 16 *** | | | |
| | **MBC** (µg/ml ) | | 16 | | | | 32 | | | |
| **20** | **MIC (µg/ml )** | | **8 - 32 *** | | | | **128** | | | **20** |
| | MBC (µg/ml ) | | / | | | | / | | | |
| **21** | **MIC (µg/ml )** | | **16 - 64 *** | | | | **32** - **64*** | | | |
| | MBC (µg/ml ) | | / | | | | / | | | |

| | | | | | | | | | | |
|---|---|---|---|---|---|---|---|---|---|---|
| wherein* MIC value read at 48 hours | | | | | | | | | | |

## Claims

1. Compounds of formula 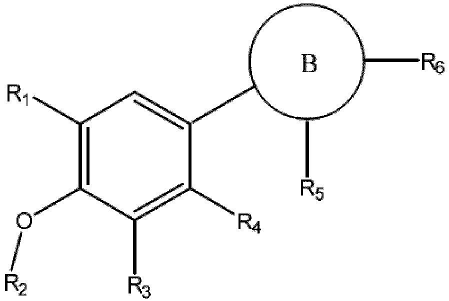 wherein
B is C₃-C₁₂ cycloakyl, or phenyl, or a C₃-C₁₃ heterocycle containing S,N,O
R1 is H, C₁-C₁₂ alkyl, C₁-C₁₂ alkenyl, C₁-C₁₂ alkynyl linear or branched, C₃-C₁₂ cycloalkyl optionally substituted with C₁-C₄ alkyl or OH, C₁-C₃ arylalkyl, phenyl, optionally substituted with C₁-C₄ alkyl, linear or branched, heterocycle containing at least one heteroatom selected from the group consisting of: N, O,S, optionally substituted with OH, NH₂
R2 is H, C₁-C₁₂ alkyl, C₁-C₁₂ alkenyl, C₁-C₁₂ alkynyl linear or branched, (CH₂)ₙ-R7, (CH₂)ₙO(CH₂)ₘO-(CH2)p-R8, (CH₂O)n CH₃, CO-R8, (CH₂)ₙ-R10, D-mannosyl, glucuronate, (SO₂)OH
R7 is OH, COOH, CONHO-R8, (CH₂)ₙO(CH₂)ₘO-R8, CN, NH₂,
R8 is H, C₁-C₁₂ alkyl, C₁-C₁₂ alkenyl, C₁-C₁₂ alkynyl linear or branched
R10 is NH₂, N-containing heterocycle, amidine, guanidine
R3 is H, OH, CHO or
R2 and R3 are linked to form a cycle comprising at least one atom of O and/or N
R4 is H
R5 is CH=NO-R14, CH=NO- (CH₂)ₙ-NH₂, (CH₂)ₙNHCO-R14,
R14 is C₁-C₅ alkyl, C₁-C₅ alkenyl, C₁-C₅ alkynyl linear or branched, C₃-C₁₂ cycloakyl, aryl, C₁-C₃ arylalkyl, (CH₂)n-COOH
R4 and R5 can be joined together to form CH=CH or CO or O or S or SO or SO₂
R6 is =CHCOOH, (CH₂)ₙCH=NOH, =CHCOOR16, CX=CYCOOH, CX=CYCN, CX=CYCOO-R16, CX=CY-tetrazolyl, CX=CYCONH₂, CX=CY-CONH-R16, CX=CYCONH-aryl, CX=CYCONHOH, CX=CYCH₂OH, C≡CCOOH, C≡CCH₂OH, C≡CCOO-R16, C≡CCONH₂, C≡CONH-R16, C=CCONH-aryl, C=CCONHOH, CX=CYCOOglycosyl, C=CCOOglycosyl,
R16 is H, C₂-C₅ alkyl, C₂-C₅ alkenyl, C₂-C₅ alkynyl linear or branched, C₃-C₁₂ cycloakyl, aryl, C₁-C₃ arylalkyl, (CH₂)n-COOH
X and Y, the same or different, are H, C₂-C4 alkyl linear or branched, halogen, CN,
n is an integer from 1 to 8
with the proviso that when R14 is CH₃, tert-butyl or (CH2)COOH, R6 is not CH=CH-COOH
or a pharmaceutically acceptable salt thereof.

2. Compounds of claim 1 for use as a medicament.

3. Compounds of claim 1 for use as antimicrobial and/or antibacterial and/or bactericide.

4. Pharmaceutical composition comprising at least one compound of claim 1 as active ingredient and pharmaceutically acceptable adjuvants and/or vehicles and/or excipients.

## Patentansprüche

1. Verbindungen der Formel 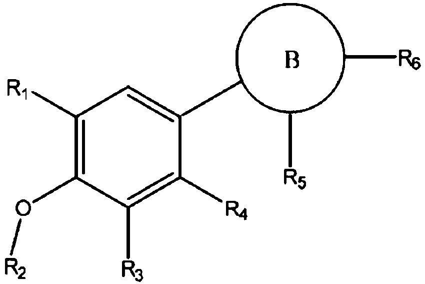 worin
B C₃-C₁₂-Cycloakyl, oder Phenyl, oder ein C₃-C₁₃-Heterozyklus, enthaltend S, N, O ist
R1 H, C₁-C₁₂-Alkyl, C₁-C₁₂-Alkenyl, C₁-C₁₂-Alkinyl, linear oder verzweigt, C₃-C₁₂-Cycloalkyl, optional substituiert mit C₁-C₄-Alkyl oder OH, C₁-C₃-Arylalkyl, Phenyl, optional substituiert mit C₁-C₄-Alkyl, linear oder verzweigt, Heterozyklus, enthaltend wenigstens ein Heteroatom, ausgewählt aus der Gruppe, bestehend aus: N, O, S, optional substituiert mit OH, NH₂ ist
R2 H, C₁-C₁₂-Alkyl, C₁-C₁₂-Alkenyl, C₁-C₁₂-Alkinyl, linear oder verzweigt, (CH₂)ₙ-R7, (CH₂)ₙO(CH₂)ₘO-(CH2)p-R8, (CH₂O)n CH₃, CO-R8,
(CH₂)ₙ-R10, D-Mannosyl, Glucuronat, (SO₂)OH ist
R7 OH, COOH, CONHO-R8, (CH₂)ₙO(CH₂)ₘO-R8, CN, NH₂ ist,
R8 H, C₁-C₁₂-Alkyl, C₁-C₁₂-Alkenyl, C₁-C₁₂-Alkinyl, linear oder verzweigt ist
R10 NH₂, N-enthaltender Heterozyklus, Amidin, Guanidin ist
R3 H, OH, CHO ist oder
R2 und R3 verknüpft sind, um einen Zyklus zu bilden, der wenigstens ein O- und/oder ein N-Atom umfasst
R4 H ist
R5 CH=NO-R14, CH=NO-(CH₂)ₙ-NH₂, (CH₂)ₙNHCO-R14 ist,
R14 C₁-C₅-Alkyl, C₁-C₅-Alkenyl, C₁-C₅-Alkinyl, linear oder verzweigt, C₃-C₁₂-Cycloakyl, Aryl, C₁-C₃-Arylalkyl, (CH₂)n-COOH ist
R4 und R5 miteinander verbunden sein können, um CH=CH oder CO oder O oder S oder SO oder SO₂ zu bilden
R6 =CHCOOH, (CH₂)ₙCH=NOH, =CHCOOR16, CX=CYCOOH, CX=CYCN, CX=CYCOO-R16, CX=CY-tetrazolyl, CX=CYCONH₂,
CX=CY-CONH-R16, CX=CYCONH-aryl, CX=CYCONHOH, CX=CYCH₂OH, C≡CCOOH, C≡CCH₂OH, C≡CCOO-R16, C≡CCONH₂,
C=CONH-R16, C=CCONH-aryl, C≡CCONHOH, CX=CYCOOglycosyl,
C=CCOOglycosyl ist,
R16 H, C₂-C₅-Alkyl, C₂-C5-Alkenyl, C₂-C₅-Alkinyl, linear oder verzweigt, C₃-C₁₂-Cycloakyl, Aryl, C₁-C₃-Arylalkyl, (CH₂)n-COOH ist
X und Y, gleich oder verschieden, H, C₂-C₄-Alkyl, linear oder verzweigt, Halogen, CN sind
n eine ganze Zahl von 1 bis 8 ist
unter der Bedingung, dass wenn R14 CH₃, tert-Butyl oder (CH2)COOH ist, R6 nicht CH=CH-COOH ist,
oder ein pharmazeutisch veträgliches Salz davon.

2. Verbindungen nach Anspruch 1 zur Verwendung als Medikament.

3. Verbindungen nach Anspruch 1 zur Verwendung als antimikrobiell und/oder antbakteriell und/oder bakterizid wirkendes Mittel.

4. Pharmazeitische Zusammensetzung umfassend wenigstens eine Verbindung nach Anspruch 1 als aktiven Bestandteil und pharmazeutisch verträgliche Hilfsstoffe und/oder Vehikel und/oder Bindemittel.

## Revendications

1. Composés de formule 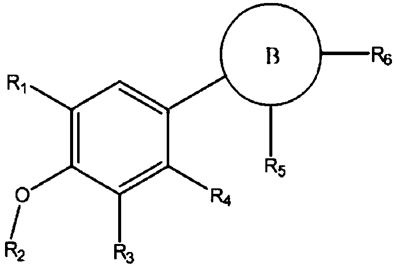 dans laquelle
B représente un groupe cycloalkyle en C₃-C₁₂, ou un groupe phényle, ou un hétérocycle en C₃-C₁₃ contenant S, N, O
R1 représente H, un groupe alkyle en C₁-C₁₂, alcényle en C₁-C₁₂, alcynyle en C₁-C₁₂, linéaire ou ramifié, cycloalkyle en C₃-C₁₂ éventuellement substitué par un groupe alkyle en C₁-C₄ ou OH, un groupe arylalkyle en C₁-C₃, un groupe phényle, éventuellement substitué par un groupe alkyle en C₁-C₄, linéaire ou ramifié, un hétérocycle contenant au moins un hétéroatome choisi parmi le groupe constitué de : N, O, S, éventuellement substitués par OH, NH₂
R2 représente H, un groupe alkyle en C₁-C₁₂, alcényle en C₁-C₁₂, alcynyle en C₁-C₁₂, linéaire ou ramifié, (CH₂)ₙ-R7, (CH₂)ₙO(CH₂)ₘO-(CH₂)p-R8, (CH₂O)n CH₃, COR8, (CH₂)ₙ-R10, D-mannosyle, glucuronate, (SO₂)OH
R7 représente OH, COOH, CONHO-R8, (CH₂)ₙO(CH₂)ₘO-R8, CN, NH₂,
R8 représente H, un groupe alkyle en C₁-C₁₂, alcényle en C₁-C₁₂, alcynyle en C₁-C₁₂, linéaire ou ramifié
R10 représente NH₂, un hétérocycle contenant de l'azote, une amidine, une guanidine
R3 représente H, OH, CHO ou
R2 et R3 sont liés pour former un cycle comprenant au moins un atome d'O et/ou de N
R4 représente H
R5 représente CH=NO-R14, CH=NO-(CH₂)ₙ-NH₂, (CH₂)ₙNHCO-R14,
R14 représente un groupe alkyle en C₁-C₅, alcényle en C₁-C₅, alcynyle en C₁-C₅, linéaire ou ramifié, cycloalkyle en C₃-C₁₂, aryle, arylalkyle en C₁-C₃, (CH₂)n-COOH
R4 et R5 peuvent être liés ensemble pour former CH=CH ou CO ou O ou S ou SO ou SO₂
R6 représente =CHCOOH, (CH₂)ₙCH=NOH, =CHCOOR16, CX=CYCOOH, CX=CYCN, CX=CYCOO-R16, CX=CY-tétrazolyle, CX=CYCONH₂, CX=CY-CONH-R16, CX=CYCONH-aryle, CX=CYCONHOH, CX=CYCH₂OH, C=CCOOH, C≡CCH₂OH, C=CCOO-R16, C≡CCONH₂, C=CONH-R16, C=CCONH-aryle, C=CCONHOH, CX=CYCOOglycosyle, C=CCOOglycosyle,
R16 représente H, un groupe alkyle en C₂-C₅, alcényle en C₂-C₅, alcynyle en C₂-C₅, linéaire ou ramifié, cycloalkyle en C₃-C₁₂, aryle, arylalkyle en C₁-C₃, (CH₂)n-COOH
X et Y, identiques ou différents, représentent H, un groupe alkyle en C₂-C₄, linéaire ou ramifié, un halogène, CN,
n est un nombre entier de 1 à 8
à condition que, lorsque R14 représente CH₃, tert-butyle ou (CH2)COOH, R6 ne représente pas CH=CH-COOH
ou un sel pharmaceutiquement acceptable de ceux-ci.

2. Composés selon la revendication 1, pour une utilisation comme médicament.

3. Composés selon la revendication 1, pour une utilisation comme antimicrobien et/ou antibactérien et/ou bactéricide.

4. Composition pharmaceutique comprenant au moins un composé selon la revendication 1 en tant que principe actif et des adjuvants et/ou véhicules et/ou excipients pharmaceutiquement acceptables.
